# EUROPEAN PATENT SPECIFICATION

(11) **EP 3 604 318 B1**
(45) Date of publication and mention of the grant of the patent: **17.01.2024**
(21) Application number: 18775752.1
(22) Date of filing: 29.03.2018
(51) Int. Cl.: C07F 9/09, A61K 6/00, C09J 4/02

(54) **PHOSPHORUS-CONTAINING COMPOUND**
PHOSPHORHALTIGE VERBINDUNG
COMPOSÉ CONTENANT DU PHOSPHORE

(30) Priority: 31.03.2017 JP 2017072679
(43) Date of publication of application: 05.02.2020
(73) Proprietor: Mitsui Chemicals, Inc., Tokyo 104-0028 (JP)
(72) Inventor: KOSUGI, Yoko, Sodegaura-shi Chiba 299-0265 (JP); MATSUMOTO, Akiko, Sodegaura-shi Chiba 299-0265 (JP); YOSHINAGA, Kazuhiko, Sodegaura-shi Chiba 299-0265 (JP)
(74) Representative: Mewburn Ellis LLP
(86) International application number: PCT/JP2018/013237
(87) International publication number: WO 2018/181711

(56) References cited:
- JP-A- S6 299 388
- JP-A- 2015 047 745
- JP-A- 2016 523 936
- US-A- 4 663 184
- US-A1- 2013 047 887
- US-A1- 2014 296 364
- US-A1- 2015 059 605

## Description

### TECHNICAL FIELD

The present invention relates to phosphorus-containing compounds, adhesive materials including the compounds, and kits including the adhesive materials.

### BACKGROUND ART

Compositions containing compounds containing a phosphate group and a (meth)acryloyl group in one molecule are known to have high bonding performance, and are widely used in industrial fields.

Attempts are being made to improve the molecular structures of such compounds containing a phosphate group and a (meth)acryloyl group, so that the performance of the compounds is enhanced (see, for example, Patent Literature 1) .

Further, compositions containing such compounds containing a phosphate group and a (meth)acryloyl group are also used as adhesive materials in the dental field, and in particular, compositions containing 10-methacryloyloxydecyl dihydrogen phosphate are widely used as dental materials (see, for example, Patent Literature 2).

However, research is still being conducted to obtain adhesive materials which exhibit completely satisfactory performance with regard to performance as adhesive materials, particularly adhesive strength as adhesive materials, and compounds to be used as raw materials for such adhesive materials.

Patent Literature 3 discloses dental adhesive compositions used for bonding dental biomaterials to hard tissue comprising a polymerizable blend of one or more acidic-methacrylate derivatives.

### CITATION LIST

### PATENT LITERATURE

Patent Literature 1: JP-W-2012-506929
Patent Literature 2: JP-A-2015-067551
Patent Literature 3: US 2013/047887

### SUMMARY OF INVENTION

### TECHNICAL PROBLEM

In view of the above-described problem, objects of the present invention are to provide adhesive materials which exhibit high adhesive strength, and compounds which can be blended with the adhesive materials.

### SOLUTION TO PROBLEM

The present inventors have conducted studies for solving the above-described problem, and as a resultant found that a cured product composed of a composition containing a (meth)acrylate compound having phosphorus-containing groups and carbamate groups has high adhesive strength. Consequently, the present invention has been completed.

The present invention includes the subject matters described below.

The present invention provides a compound represented by the general formula (1) below, in which the core (X) below and the terminal group (Y1) below are bonded to each other:
[Chem. 1]

X (Y 1) n¹ (1)

(in the general formula (1), n¹ represents the number of terminal groups (Y1) bonded to the core (X), and n¹ is equal to the valence of the core (X) ; and the terminal group (Y1) is a phosphorus-containing group represented by the general formula (2) below, a phosphorus-containing group represented by the general formula (3) below, a (meth)acryloyl group-containing group (Y2) represented by the general formula (4) below, a (meth)acryloyl group, a C₁₋₂₀ hydrocarbon group or a hydrogen atom, and a plurality of terminal groups (Y1) may be the same as or different from each other, with the proviso that among all the terminal groups (Y1) in the compound represented by the general formula (1), one or more terminal groups are phosphorus-containing groups represented by the general formula (2) or phosphorus-containing groups represented by the general formula (3), and one or more terminal groups are (meth)acryloyl group-containing groups (Y2)); 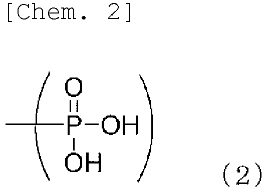 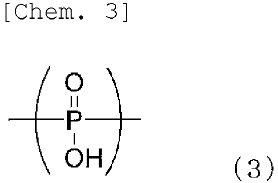 (in the general formula (3), one end of the group is bonded to the core (X), and the other end of the group is bonded to the core (X) present in another compound represented by the general formula (1)); and 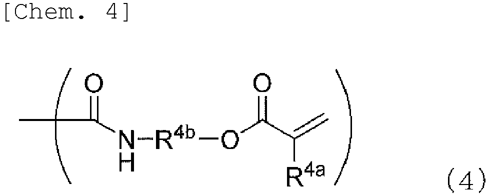 (in the general formula (4), R^{4a} represents a hydrogen atom or a methyl group, R^{4b} represents a C₂₋₆ linear alkylene group or a C₂₋₆ linear oxyalkylene group, and the linear alkylene group or the linear oxyalkylene group is optionally substituted with a C₁₋₆ alkyl group or a (meth)acryloyloxymethylene group in place of a hydrogen atom); wherein the core (X) is at least one selected from the group consisting of groups represented by the general [0013] formulas (5a) to (5h).

In some embodiments the terminal group (Y1) is a phosphorus-containing group represented by the general formula (2), a phosphorus-containing group represented by the general formula (3), a (meth)acryloyl group-containing group (Y2) represented by the general formula (4), or a hydrogen atom.

In some embodiments the terminal group (Y1) is a phosphorus-containing group represented by the general formula (2), a phosphorus-containing group represented by the general formula (3), or a (meth)acryloyl group-containing group (Y2) represented by the general formula (4). 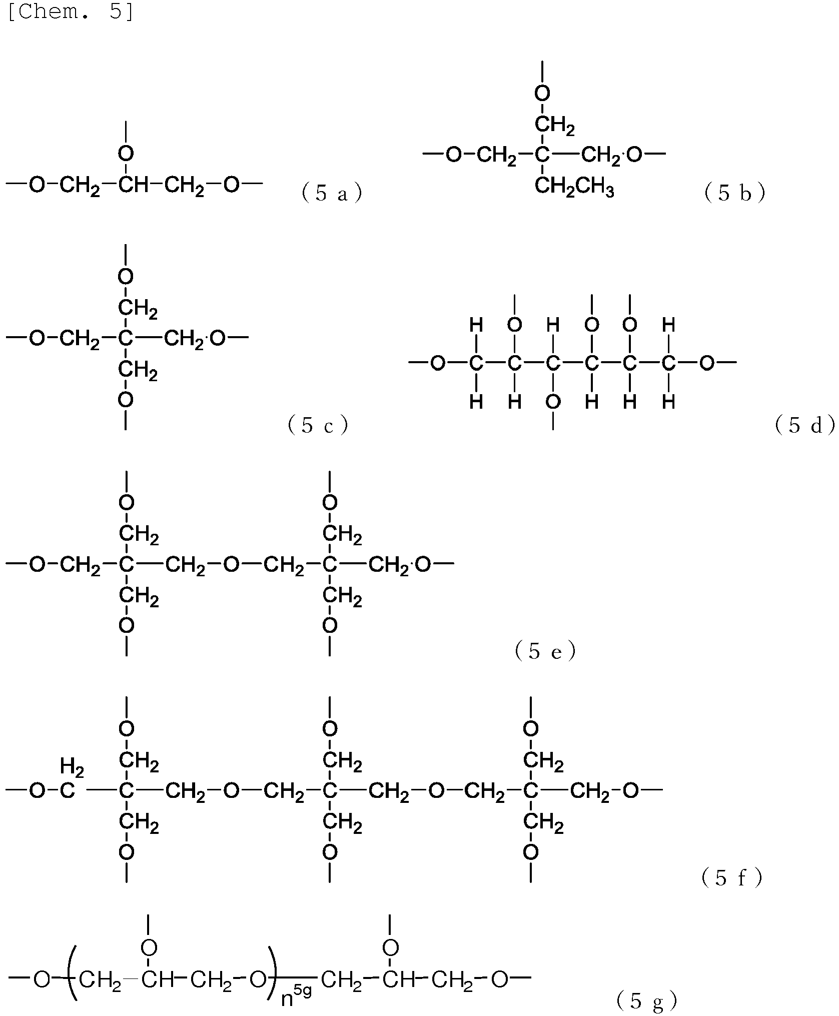 (n^{5g} in the general formula (5g) is an integer of 1 to 40)

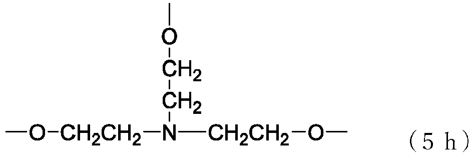

In some embodiments the (meth)acryloyl group-containing group (Y2) is at least one selected from the group consisting of groups represented by the general formulas (4a) to (4f).

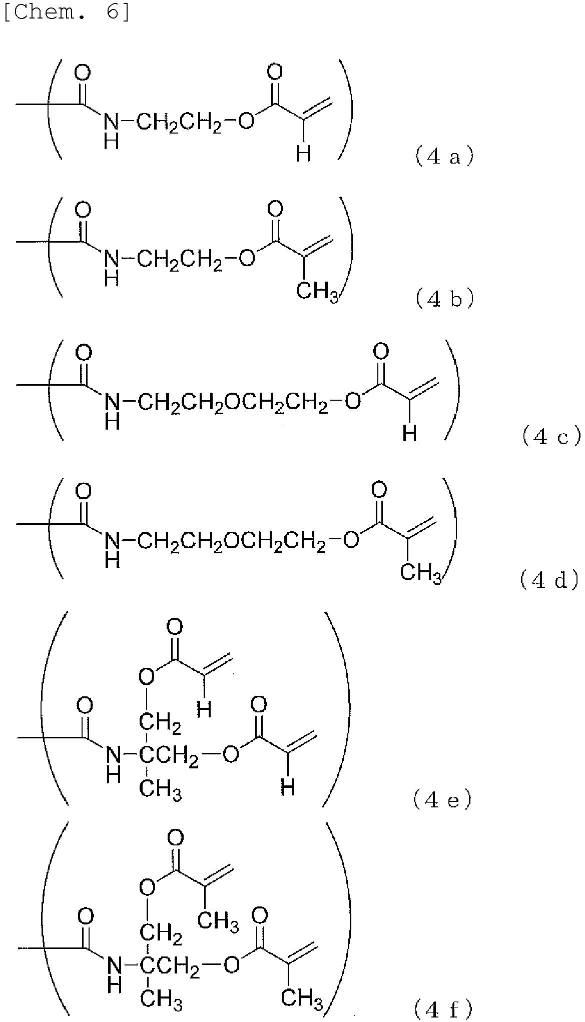

The present invention also provides a composition comprising the compound described above.

In some embodiments the composition is negative in a reverse mutation test.

The present invention also provides an adhesive material comprising the compound described above.

In some embodiments the adhesive material is negative in a reverse mutation test.

The present invention also provides a kit comprising the adhesive material described above.

### ADVANTAGEOUS EFFECTS OF INVENTION

The compounds of the invention are suitable for adhesive materials, and adhesive materials including the compounds exhibit high adhesive strength.

### DESCRIPTION OF EMBODIMENTS

Hereinafter, the present invention will be described in detail. Herein, the "(meth)acryl" means acryl or methacryl, and for example, the "(meth)acrylic acid" means methacrylic acid or acrylic acid. Similarly, the "(meth)acryloyl" means "acryloyl" or "methacryloyl", and the "(meth)acrylate" means "acrylate" or "methacrylate".

### [Compounds]

The compounds of the present invention are compounds represented by the general formula (1) below, in which the core (X) below and the terminal group (Y1) below are bonded to each other (hereinafter, also referred to as compounds (1)).
[Chem. 7]

X (Y 1) n¹ (1)

In the general formula (1), n¹ represents the number of terminal groups (Y1) bonded to the core (X), n¹ is equal to the valence of the core (X), and n¹ is an integer of not less than 3, preferably an integer of 3 to 12, more preferably an integer of 3 to 8.

### [Core (X)]

The core (X) is a polyvalent organic group having a valence of not less than 3 containing an oxygen atom or a nitrogen atom in which an atom bonded to the terminal group (Y1) is the oxygen atom or the nitrogen atom. The oxygen atom or nitrogen atom bonded to the terminal group (Y1) is bonded to a methylene group or a divalent aromatic carbon group in addition to the terminal group (Y1).

The core (X) is at least one selected from the groups represented by the general formulas (5a) to (5h) below. 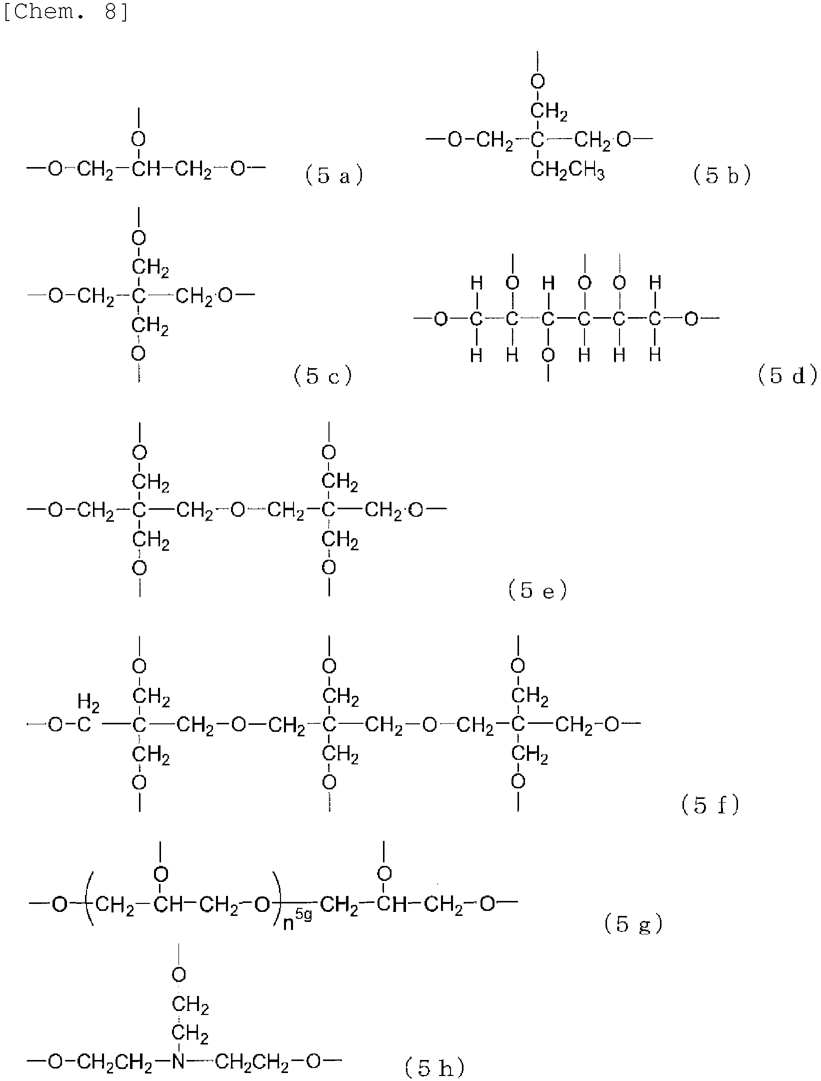 n^{5g} in the general formula (5g) is an integer of 1 to 40, preferably 1 to 20, more preferably 1 to 10, still more preferably 1 to 5.

### [Terminal group (Y1)]

The terminal group (Y1) is a phosphorus-containing group represented by the general formula (2), a phosphorus-containing group represented by the general formula (3), a (meth)acryloyl group-containing group (Y2) represented by the general formula (4), a (meth)acryloyl group, a C₁₋₂₀ hydrocarbon group or a hydrogen atom, and a plurality of terminal groups (Y1) may be the same as or different from each other, with the proviso that among all the terminal groups (Y1) in the compound represented by the general formula (1), one or more terminal groups are phosphorus-containing groups represented by the general formula (2) or phosphorus-containing groups represented by the general formula (3), and one or more terminal groups are (meth)acryloyl group-containing groups (Y2). 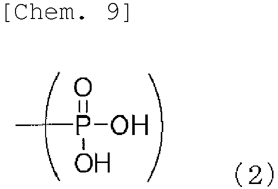 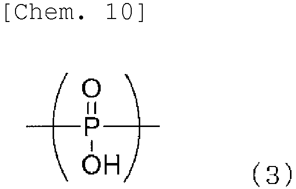 In the general formula (3), one end of the group is bonded to the core (X), and the other end of the group is bonded to the core (X) present in another compound represented by the general formula (1). 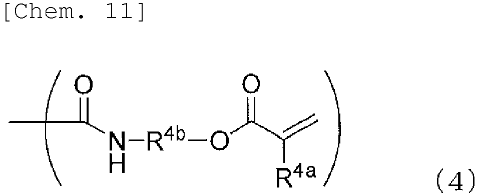 In the general formula (4), R^{4a} represents a hydrogen atom or a methyl group, R^{4b} represents a C₂₋₆ linear alkylene group or a C₂₋₆ linear oxyalkylene group, and the linear alkylene group or the linear oxyalkylene group is optionally substituted with a C₁₋₆ alkyl group or a (meth)acryloyloxymethylene group in place of a hydrogen atom. Examples of the (meth)acryloyl group-containing group (Y2) represented by the general formula (4) include groups represented by the general formulas (4a) to (4f) below.

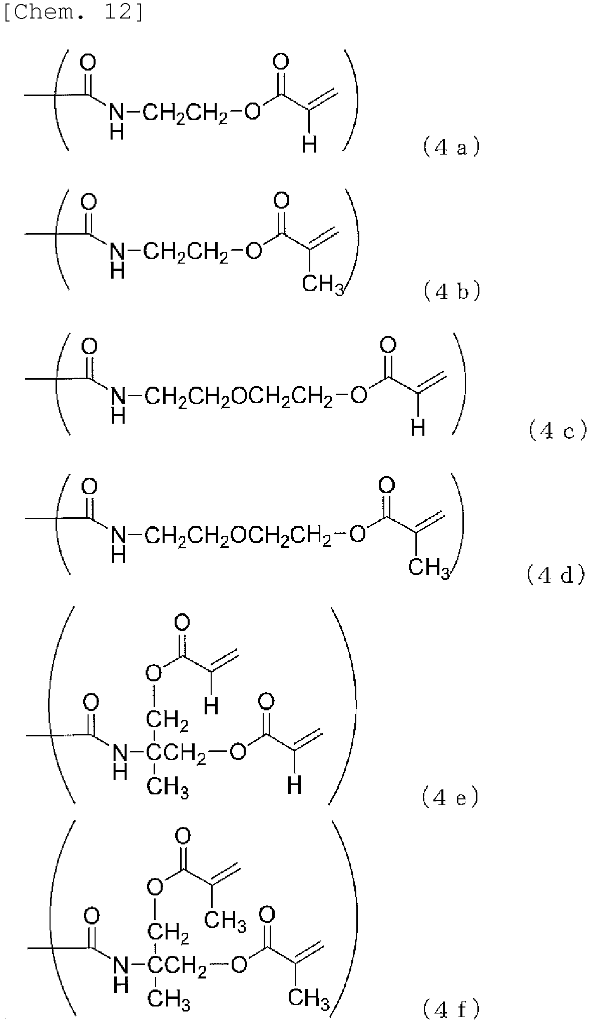

The terminal group (Y1) is a phosphorus-containing group represented by the general formula (2), a phosphorus-containing group represented by the general formula (3), a (meth)acryloyl group-containing group (Y2) represented by the general formula (4), a (meth)acryloyl group, a C₁₋₂₀ hydrocarbon group or a hydrogen atom as described above, preferably a phosphorus-containing group represented by the general formula (2), a phosphorus-containing group represented by the general formula (3), a (meth)acryloyl group-containing group (Y2) represented by the general formula (4) or a hydrogen atom, more preferably a phosphorus-containing group represented by the general formula (2), a phosphorus-containing group represented by the general formula (3) or a (meth)acryloyl group-containing group (Y2) represented by the general formula (4).

Examples of the compounds of the invention include compounds represented by the general formulas (1a) to (1k). 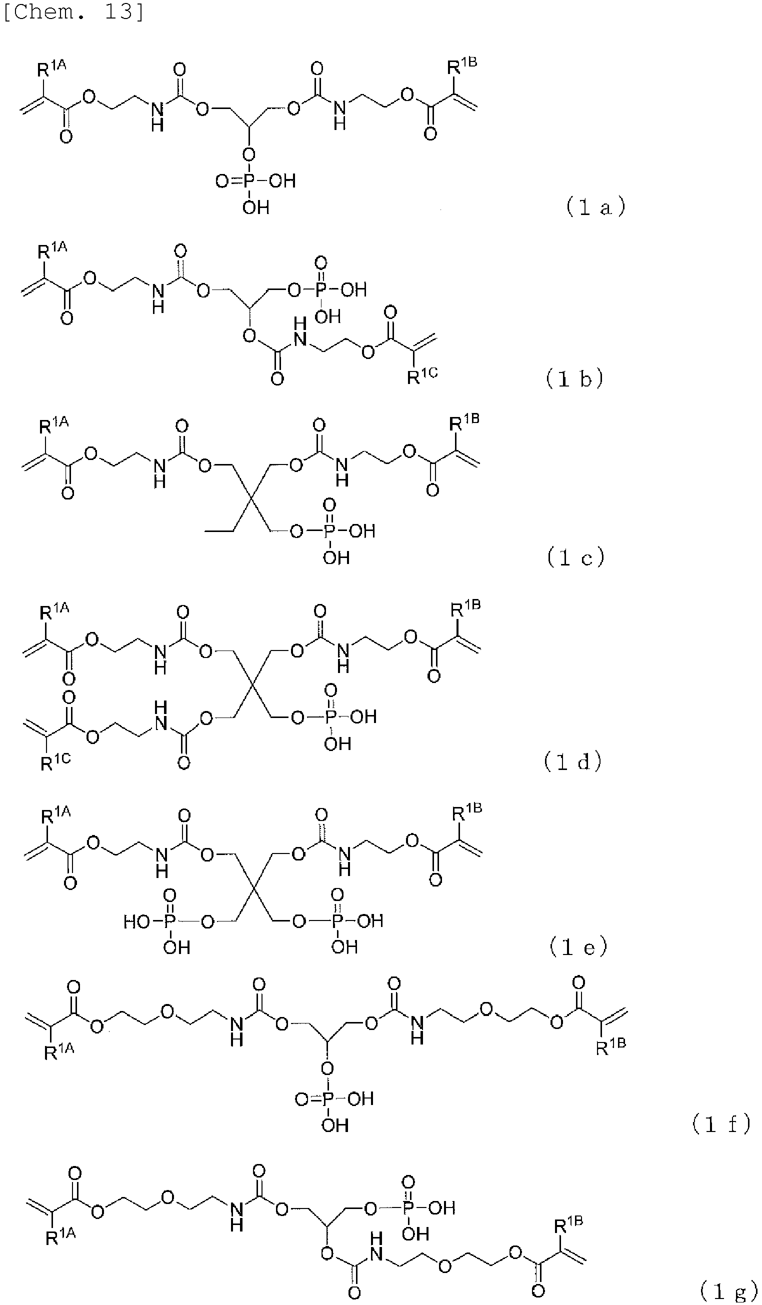 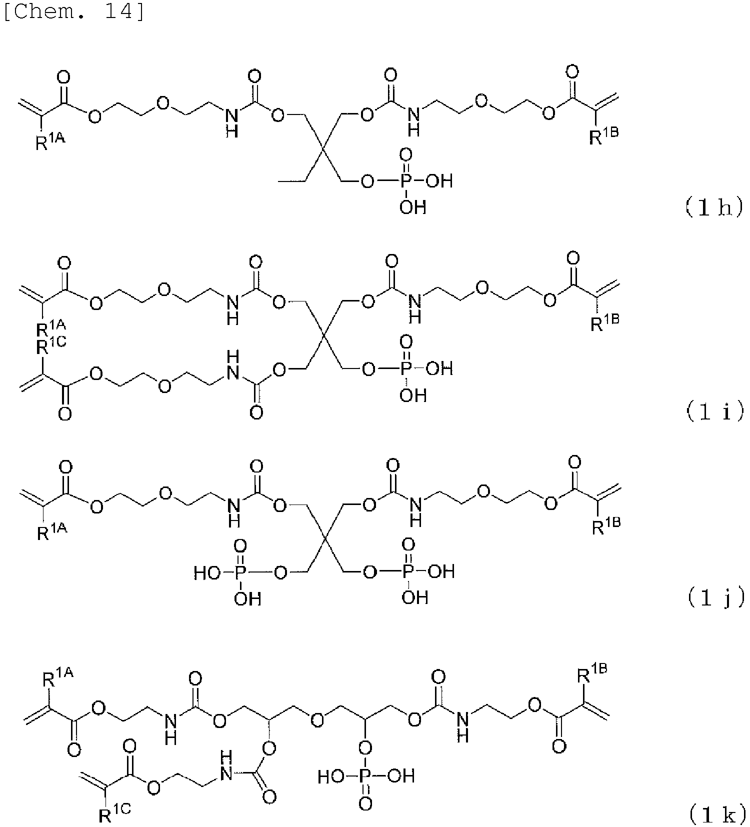 In the general formulas (1a) to (1k), R^{1A}, R^{1B} and R^{1C} are hydrogen atoms or methyl groups.

### [Method for producing compound (1)]

The compounds (1) of the invention can be produced by a known method.

For example, a corresponding hydroxyl group-containing compound or amino group-containing compound, preferably a corresponding hydroxyl group-containing compound is reacted with a known phosphorylating agent (for example phosphorus pentaoxide, phosphorus pentachloride, phosphorus trioxide, phosphorus oxychloride or phosphorus pentasulfide) by a known method to form hydroxyl groups or amino groups, preferably hydroxyl groups, present in the compound into phosphoric acid esters or phosphoric acid amides.

The hydroxyl group or amino group-containing compounds corresponding to the compounds (1) may be compounds represented by the general formula (6) below, in which the core (X) below and the terminal group (Y3) below are bonded to each other.
[Chem. 15]

X(Y3)n⁶ (6)

In the general formula (6), n⁶ represents the number of terminal groups (Y3) bonded to the core (X), n⁶ is equal to the valence of the core (X), and n⁶ is an integer of not less than 3, preferably an integer of 3 to 12, more preferably an integer of 3 to 8. The core (X) is as described for the general formula (1).

### [Terminal group (Y3)]

The terminal group (Y3) is a (meth)acryloyl group-containing group (Y2) represented by the general formula (4), a (meth) acryloyl group, a C₁₋₂₀ hydrocarbon group or a hydrogen atom, preferably a (meth)acryloyl group-containing group (Y2) represented by the general formula (4), a (meth)acryloyl group or a hydrogen atom, more preferably (meth)acryloyl group-containing group (Y2) represented by the general formula (4) or a hydrogen atom. A plurality of terminal groups (Y3) may be the same as or different from each other, with the proviso that among all the terminal groups (Y3) in the compound represented by the general formula (6), one or more terminal groups are (meth)acryloyl group-containing groups (Y2), and one or more terminal groups are hydrogen atoms. Preferably, two or more terminal groups are (meth)acryloyl group-containing groups (Y2), and one or more terminal groups are hydrogen atoms.

In production of the compounds (1) of the invention by the above method, all the hydroxyl groups or amino groups in the corresponding hydroxyl group or amino group-containing compound may be formed into phosphoric acid esters or phosphoric acid amides, or some hydroxyl groups or amino groups may remain. The products thus obtained can be used for adhesive materials when containing the compounds (1).

Further, in production of the compounds of the invention by the above method, one ester group or amide group may be introduced, or two ester groups or amide groups may be introduced on one phosphorus atom. The products thus obtained can be used for adhesive materials when containing the compounds (1).

### [Specific examples of compounds of the general formula (6)]

Examples of the compounds of the general formula (6) include (meth)acryloyl group-containing group (Y2)-containing alcohols represented by the general formulas (11a) to (11k). 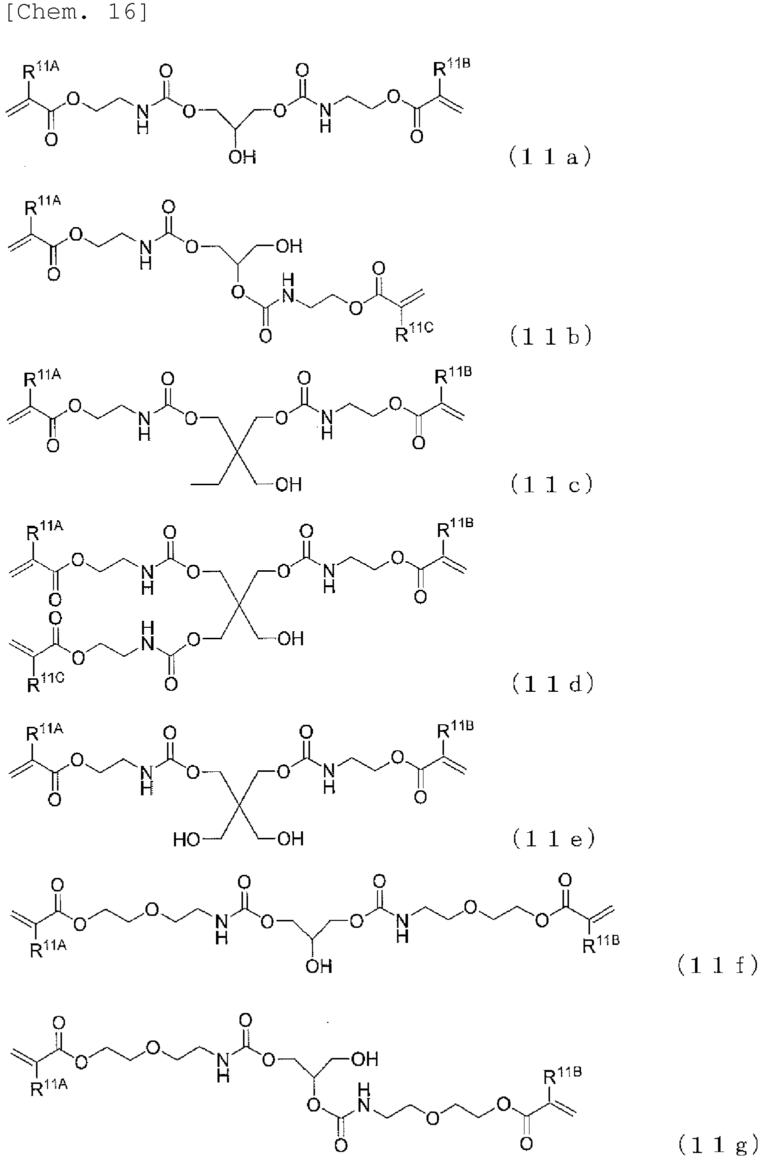 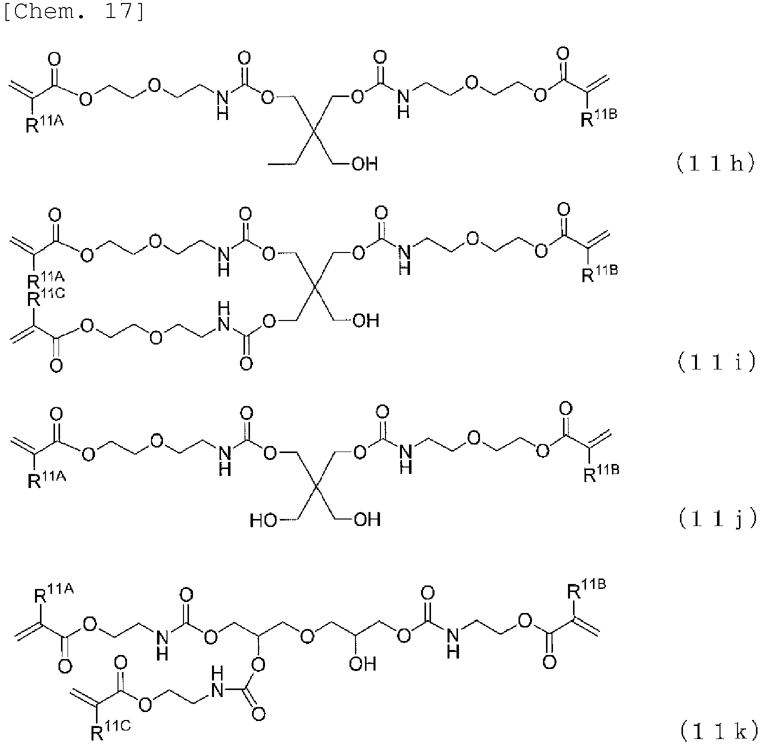 In the general formulas (11a) to (11k), R^{11A}, R^{11B} and R^{11C} are hydrogen atoms or methyl groups.

### [Composition containing compound (1)]

The composition according to the invention optionally contain polymerizable monomers other than the compounds (1) of the invention (for example (meth)acrylate group-containing monomers other than the compounds (1) of the invention) which can be blended with the later-described adhesive material.

In the composition according to the invention, for example, the content of the compound (1) of the invention may be not less than 1.0 mass% (e.g. not less than 10 mass%, not less than 50 mass%, not less than 80 mass% or not less than 90 mass%), and may be not more than 100 mass% (e.g. not more than 99 mass%, not more than 90 mass%, not more than 80 mass%, not more than 50 mass% or not more than 10 mass%) based on the total amount of the composition.

### [Reverse mutation test]

The composition according to the invention is preferably negative in a reverse mutation test. The reverse mutation test (Ames test) means a test for examining the mutagenicity of a composition using microorganisms. The reverse mutation test in the present invention is conducted by the following method.

Specifically, the reverse mutation test is conducted under a fluorescent lamp with an ultraviolet absorbing film and/or a LED in accordance with the following procedure.

First, to a sterilized test tube are added 0.1 mL of a test composition solution having dimethyl sulfoxide (DMSO) as a medium, and 0.5 mL of a 0.1 M phosphate buffer solution (pH 7.4) in the case where metabolic activation is not performed, or 0.5 mL of the later-described S9mix in the case where metabolic activation is performed. Thereafter, 0.1 mL of the later-described bacterial suspension is added, and the resulting mixture is mixed. The resulting mixture is preincubated at about 100 rpm for 20 minutes at 37°C, 2 mL of the later-described top agar is then added, and the resulting mixture is mixed, and overlaid on the later-described minimal glucose agar plate medium (five or more doses). After it is confirmed that the mixture has been overlaid and solidified, the minimal glucose agar plate medium is turned upside down, and culture is performed at 37°C for 48 hours. Whether the result is negative or positive is determined on the cultured plate. When a negative control substance is subjected to the test, DMSO is used as the negative control substance, and 0.1 mL of the medium is added instead of the test composition solution in the above-described process.

After completion of the culture, the number of revertant colonies on each plate is measured.

Regarding the criterion for determining whether the result is negative or positive, it is determined that the test composition is negative when the average number of colonies with the test composition is equal to or less than two times the average number of colonies with only the negative control substance for all strains, at all doses and in both the cases where metabolic activation is not performed and metabolic activation is performed.

In the reverse mutation test, the doses of a test substance present in the test composition solution (specifically, the compound (1) present in the composition) are adjusted so that a maximum dose of 5000 µg per plate is followed by five or more doses descending in a geometric progression with a common ratio of 2 to 4.

The strain to be used is Salmonella typhimurium TA 100 or TA 1535, or Escherichia coli WP2uvrA, which are a base pair substitution mutant strain, or Salmonella typhimurium TA 98 or TA 1537, which are a frameshift mutant strain.

The minimal glucose agar plate medium to be used is Tesmedia AN Medium (manufactured by Oriental Yeast Co., Ltd., for testing of mutagenicity).

The number of the minimal glucose agar plate media per dose is not less than 2 for the negative control substance, and not less than 2 for the test composition.

The case where metabolic activation is performed means that S9mix (a rat liver microsome fraction containing a coenzyme) is added together with a test substance, and the case where metabolic activation is not performed means that S9mix is not added. Specifically, the S9mix has the composition of S9 (a supernatant fraction of a liver homogenate centrifuged at 9000 × g): 0.1 mL, MgCl₂: 8 umol, KCl: 33 µmol, glucose-6-phosphoric acid: 5 µmol, NADPH: 4 µmol, NAPH: 4 µmol and sodium phosphate buffer solution (pH 7.4): 100 umol.

The top agar to be used is a mixture obtained by subjecting an amino acid solution (0.5 mmol/L L-histidine, 0.5 mmol/L D-biotin and 0.5 mmol/L L-tryptophane) to filtration sterilization, subjecting a soft agar solution (0.6% (w/v) agar (Bacto-Agar) and 0.5% (w/v) sodium chloride) to high-pressure steam sterilization at 121°C for 20 minutes, and mixing the amino acid solution and the melted soft agar solution at a volume ratio of 1:10.

In preparation of each bacterial suspension, the bacterial concentration is adjusted to not less than 1 × 10⁹ bacteria per mL for each bacterium. For culture of each bacterium, a nutrient broth culture is used. The nutrient broth culture is prepared by dissolving Nutrient Broth No. 2 (Oxoid, Nutrient Broth No. 2) in purified water to a concentration of 2.5 wt%, and subjecting the resulting solution to high-pressure steam sterilization at 121°C for 20 minutes.

### [Cytotoxicity test by NRU method]

The composition according to the invention may allow the relative cell survival rate to fall within a certain range in a cytotoxicity test by an NRU method using the later-described Balb/3T3 cells. The cytotoxicity test is conducted by the following method.

Balb/3T3 cells (Balb/3T3 clone A31 cells (mouse skin-derived fibroblast cells)) are seeded at a density of 10000 cells per well in a 96-well plate, and precultured for 25 hours, the medium in each well is then removed, 0.1 mL of a test solution containing a test composition or a negative control solution is added to the cells, and the cells are cultured in a CO₂ incubator for 24 hours. Here, 12 wells are used for the negative control solution, and 6 wells are used for the test solution. After the culture, each well is observed under a microscope to confirm growth of the cells, the culture in each well is removed, and washing is performed with 0.15 mL of PBS. After the washing, 0.1 mL of an NR culture is added to each well, and the cells are cultured in a CO₂ incubator for 3 hours to perform staining. After the culture, the culture in each well is removed, and washing is performed with 0.15 mL of PBS. 0.15 ml of an NR-redissolving solution is added to each well, and shaken with a plate shaker for 10 minutes. Neutral red (NR) is dissolved in the NR-redissolving solution, the absorbance of the solution in each well is then measured at 540 nm, and the average value of the absorbances is determined. The absorbance of a solution in a well containing the test solution is calculated as a relative value against the absorbance of a solution in a well containing the negative control solution, with the latter absorbance set to 100, and the thus-obtained value is defined as a relative cell survival rate (%) of a test composition containing a test substance (a composition comprising the compound (1)).

For preparation of a test solution containing a test composition, a test composition is added to DMSO, and the resulting mixture is then diluted with DMSO to prepare a DMSO solution. Thereafter, 10 µL of the above-described DMSO solution is added per 2 mL of the later-described D05 culture, and the resulting mixture is stirred and mixed to prepare a test solution. The test solution is adjusted so that the test composition solution contains a test substance at a predetermined concentration (specifically, the composition contains the compound (1) at a predetermined concentration).

The negative control solution is prepared by adding DMSO to the D05 culture to a concentration of 0.5 v/v%.

The D05 culture is D-MEM (Dulbecco's Modified Eagle's Medium 9, Cat No. 048-30275, containing 584 mg/L of glutamine and 5.958 g/L of HEPES) which contains 5 vol% of calf serum, 1 mmol/L sodium pyruvate and 1 vol% of a penicillin-streptomycin-amphotericin B suspension.

The D10 culture is D-MEM containing 10 vol% of calf serum, 1 vol% of a penicillin-streptomycin-amphotericin B suspension and 1 vol% of a 100 mmol/L sodium pyruvate solution.

The preculture of the Balb/3T3 cells is performed in the following manner: cells in the logarithmic growth phase are isolated using trypsin-EDTA, a cell suspension having a cell concentration of 1 × 10⁵ cells/mL is then prepared using a D05 culture, and 0.1 mL of the cell suspension is then dispensed and seeded in a 96-well plate (1 × 10⁴ cells/well), and left standing in a CO₂ incubator for 25 hours.

The NR culture is a culture obtained by mixing an NR (neutral red) stock solution and a D10 culture at a ratio of 1:79, leaving the resulting mixture overnight at 37°C, and then removing the NR crystal by filtration with a filter. The NR (neutral red) stock solution is a 0.4% (w/v) aqueous solution of neutral red (NR) (manufactured by Wako Pure Chemical Industries, Ltd.). The NR-dissolving solution is a solution obtained by mixing acetic acid, ethanol and water at a ratio of 1:50:49. The NR-dissolving solution is prepared within an hour before use.

The concentration of the test substance of the test composition in the test solution may be, for example, 0.00164 mg/mL, 0.00410 mg/mL, 0.0102 mg/mL, 0.0256 mg/mL, 0.0640 mg/mL, 0.160 mg/mL, 0.400 mg/mL or 1.00 mg/mL.

When the concentration of the test substance of the test composition in the test solution is 0.00164 mg/mL, 0.00410 mg/mL, 0.0102 mg/mL, 0.0256 mg/mL, 0.0640 mg/mL, 0.160 mg/mL, 0.400 mg/mL or 1.00 mg/mL, the relative cell survival rate in the NRU method using the Balb/3T3 cells may be not less than 0.01% (e.g. not less than 0.05%, not less than 0.1%, not less than 0.5%, not less than 1.0%, not less than 5.0%, not less than 10%, not less than 20%, not less than 30%, not less than 40%, not less than 500, not less than 60%, not less than 70%, not less than 80%, not less than 90%, not less than 95% or not less than 99%), and may be not more than 100% (e.g. not more than 99%, not more than 95%, not more than 90%, not more than 80%, not more than 70%, not more than 60%, not more than 50%, not more than 40%, not more than 30%, not more than 20%, not more than 10%, not more than 5.0%, not more than 1.0%, not more than 0.5%, not more than 0.1% or not more than 0.050).

### [Cell test by WST method]

The composition according to the invention may allow the relative cell growth rate to fall within a certain range in a cell test by a WST method using the Balb/3T3 cells. The cell test is conducted by the following method.

Balb/3T3 cells (Balb/3T3 clone A31 cells (mouse skinderived fibroblast cells)) are seeded at a density of 2000 cells per well in a 96-well plate, and precultured for 24 hours, the culture in each well is then removed, 0.1 mL of a test solution containing a test composition or a negative control solution is added to the cells, and the cells are cultured in a CO₂ incubator for 48 hours. Here, 6 wells are used for the negative control solution, and 3 wells are used for the test solution.

After the culture, the test solution or the negative control solution is discarded, washing is performed with PBS, 0.2 mL of the later-described DMEM culture containing a 10% WST-8 reagent is added to each well, and color reaction is carried out in a CO₂ incubator for 2 hours. The absorbance of the solution in the well after the reaction is measured at 450 nm and 650 nm by a microplate reader. A value obtained by subtracting the 650 nm-absorbance from the 450 nm-absorbance of the solution in each well is defined as the absorbance for each well, and with a negative value set to 0 if any, the average value of the absorbances is determined. A value obtained by dividing the average absorbance of the solution in wells containing the test solution by the average absorbance of the solution in wells containing the negative control solution is defined as a relative cell growth rate (%) of a test composition containing a test substance (a composition comprising the compound (1)).

For preparation of a test solution containing a test composition, a test composition is added to DMSO, and the resulting mixture is then diluted with DMSO to prepare a DMSO solution. Thereafter, 5 µL of the solution diluted with DMSO solution is added per mL of a DMEM culture to prepare a test solution. The test solution is adjusted so that the test composition solution contains a test substance at a predetermined concentration (specifically, the composition contains the compound (1) at a predetermined concentration).

The negative control solution is prepared by adding DMSO to the DMEM culture to a concentration of 0.5 v/v%.

The DMEM culture is Dulbecco's Modified Eagle's Medium (D-MEM) containing 10 vol% of calf serum and 1 vol% of a penicillin-streptomycin-amphotericin B suspension (× 100).

The preculture of the Balb/3T3 cells is performed in the following manner: Balb/3T3 clone A31 cells in the logarithmic growth phase are isolated using 0.25% trypsin-1mM EDTA, a cell suspension having a cell concentration of 20000 cells/mL is then prepared using a DMEM culture, and 0.1 mL of the cell suspension is then dispensed and seeded in a 96-well plate (2000 cells/well), and left standing in a CO₂ incubator for 24 hours.

The concentration of the test substance of the test composition in the test solution may be, for example, 0.00164 mg/mL, 0.00410 mg/mL, 0.0102 mg/mL, 0.0256 mg/mL, 0.0640 mg/mL, 0.160 mg/mL, 0.400 mg/mL or 1.00 mg/mL.

When the concentration of the test substance of the test composition in the test solution is 0.00164 mg/mL, 0.00410 mg/mL, 0.0102 mg/mL, 0.0256 mg/mL, 0.0640 mg/mL, 0.160 mg/mL, 0.400 mg/mL or 1.00 mg/mL, the relative cell growth blocking rate in the WST method using the Balb/3T3 cells may be not less than 0.001% (e.g. not less than 0.01%, not less than 0.05%, not less than 0.1%, not less than 0.5%, not less than 1.0%, not less than 5.0%, not less than 10%, not less than 20%, not less than 300, not less than 40%, not less than 50%, not less than 60%, not less than 70%, not less than 80%, not less than 90%, not less than 95% or not less than 99%), and may be not more than 100% (e.g. not more than 99%, not more than 95%, not more than 90%, not more than 80%, not more than 70%, not more than 600, not more than 50%, not more than 40%, not more than 30%, not more than 20%, not more than 10%, not more than 5.0%, not more than 1.0%, not more than 0.5%, not more than 0.1%, not more than 0.05% or not more than 0.01%).

### [Adhesive materials containing compound (1)]

The compound (1) of the invention is suitable as a raw material for adhesive materials. By blending the compound (1) of the invention with components other than the compounds (1) of the invention (for example polymerizable monomers other than the compounds (1) of the invention ((meth)acrylate group-containing monomers other than the compounds (1) of the invention, monomers containing epoxy groups )), for example, an adhesive material can be produced.

### [(Meth)acrylate group-containing monomers other than compounds (1) of the invention]

Examples of the components other than the compounds (1) of the invention may include (meth)acrylate group-containing monomers other than the compounds (1) of the invention.

The (meth)acrylate group-containing monomer other than the compounds (1) of the invention contains one or more (meth)acrylate groups in the molecule. The number of polymerizable groups present may be 1, or not less than 2.

The (meth)acrylate group-containing monomer other than the compounds (1) of the invention may be composed of one compound, or composed of a mixture of two or more compounds.

Examples of the (meth)acrylate group-containing monomers other than the compounds (1), which have only one polymerizable group, include monomers represented by the general formula (21) below. 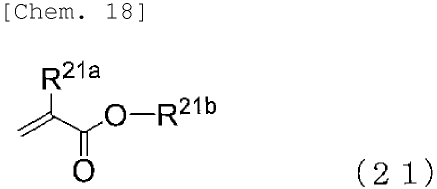 In the general formula (21), R^{21a} represents hydrogen or a methyl group, and R^{21b} represents a C₁₋₂₀ monovalent organic group which may contain oxygen or nitrogen.

Examples of the monovalent organic groups include hydrocarbon groups, for example, C₁₋₂₀ acyclic hydrocarbon groups such as alkyl groups, alkenyl groups and alkynyl groups, and C₁₋₂₀ cyclic hydrocarbon groups such as cycloalkyl groups, cycloalkenyl groups, cycloalkynyl groups and aryl groups; and C₁₋₂₀ oxygen-containing hydrocarbon groups such as those groups corresponding to the above hydrocarbon groups except that oxygen is introduced between at least part of the carbon atoms forming carbon-carbon bonds (but oxygen atoms are not inserted contiguously), for example, alkoxyalkyl groups, alkoxyalkylene glycol groups and tetrahydrofurfuryl groups. The C₁₋₂₀ cyclic hydrocarbon groups may have acyclic hydrocarbon moieties. Further, the acyclic hydrocarbon moieties present in these groups may be linear or branched.

In the case where the C₁₋₂₀ hydrocarbon groups or the C₁₋₂₀ oxygen-containing hydrocarbon groups contain linear alkylene moieties, at least one of the methylene groups in such moieties may be substituted by an ester bond, an amide bond, a carbonate bond, a urethane bond (a carbamoyl group) or a urea bond (but the methylene groups are not substituted contiguously).

Further, hydrogen atoms present in the organic groups such as the C₁₋₂₀ hydrocarbon groups and the C₁₋₂₀ oxygen-containing hydrocarbon groups may be substituted by acid groups such as carboxyl groups and phosphate groups, and functional groups such as hydroxyl groups, amino groups and epoxy groups.

Examples of the methacryloyl-containing compounds represented by the general formula (21) include methyl methacrylate, ethyl methacrylate, propyl methacrylate, butyl methacrylate, hexyl methacrylate, cyclohexyl methacrylate, ethoxydiethylene glycol methacrylate, methoxytriethylene glycol methacrylate, phenoxyethyl methacrylate, 2-hydroxyethyl methacrylate, 2-hydroxypropyl methacrylate, 2-hydroxybutyl methacrylate, 2-hydroxy-3-phenoxypropyl methacrylate, 4-hydroxybutyl methacrylate and 1,4-cyclohexanedimethanol monomethacrylate.

Examples of the methacryloyl-containing compounds represented by the general formula (21) include methyl acrylate, ethyl acrylate, propyl acrylate, butyl acrylate, hexyl acrylate, cyclohexyl acrylate, ethoxydiethylene glycol acrylate, methoxytriethylene glycol acrylate, phenoxyethyl acrylate, 2-hydroxyethyl acrylate, 2-hydroxypropyl acrylate, 2-hydroxybutyl acrylate, 2-hydroxy-3-phenoxypropyl acrylate, 4-hydroxybutyl acrylate and 1,4-cyclohexanedimethanol monoacrylate.

Examples of the (meth)acrylate group-containing monomers other than the compounds (1) of the invention, which have two or more polymerizable groups, include monomers represented by the general formula (22) below. 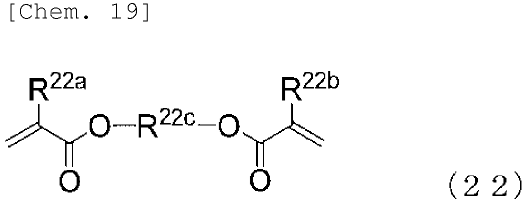 In the general formula (22), R^{22a} and R^{22b} each represent hydrogen or a methyl group and may be the same as or different from each other; and R^{22c} represents a C₁₋₄₀ divalent organic group which may contain oxygen or nitrogen.

Examples of the divalent organic groups include hydrocarbon groups, for example, C₁₋₄₀ acyclic hydrocarbon groups such as alkylene groups, alkenylene groups and alkynylene groups, and C₁₋₄₀ cyclic hydrocarbon groups such as cycloalkylene groups, cycloalkenylene groups, cycloalkynylene groups and arylene groups; and C₁₋₄₀ oxygen-containing hydrocarbon groups such as those groups corresponding to the above hydrocarbon groups except that oxygen is introduced between at least part of the carbon atoms forming carbon-carbon bonds (but oxygen atoms are not inserted contiguously), for example, oxyalkylene groups. The C₁₋₄₀ cyclic hydrocarbon groups may have acyclic hydrocarbon moieties. Further, the acyclic hydrocarbon moieties present in these groups may be linear or branched.

In the case where the C₁₋₄₀ hydrocarbon groups or the C₁₋₄₀ oxygen-containing hydrocarbon groups contain linear alkylene moieties, at least one of the methylene groups in such moieties may be substituted by an ester bond, an amide bond, a carbonate bond, a urethane bond (a carbamoyl group) or a urea bond (but the methylene groups are not substituted contiguously).

Further, hydrogen atoms present in the organic groups such as the C₁₋₄₀ hydrocarbon groups and the C₁₋₄₀ oxygen-containing hydrocarbon groups may be substituted by acid groups such as carboxyl groups and phosphate groups, functional groups such as hydroxyl groups, amino groups and epoxy groups, and polymerizable groups such as acryloyl groups and methacryloyl groups.

Among the monomers represented by the general formula (22), some preferred monomers are those monomers in which R^{22c} is a linear alkylene group having 2 to 20 carbon atoms, preferably 4 to 12 carbon atoms.

Examples of the compounds which correspond to the above preferred monomers and have methacryloyl groups include 1,4-butanediol dimethacrylate, 1,6-hexanediol dimethacrylate, 1,8-octanediol dimethacrylate, 1,9-nonanediol dimethacrylate and 1,10-decanediol dimethacrylate.

Examples of the compounds which correspond to the above preferred monomers and have acryloyl groups include 1,4-butanediol diacrylate, 1,6-hexanediol diacrylate, 1,8-octanediol diacrylate, 1,9-nonanediol diacrylate and 1,10-decanediol diacrylate.

Among the monomers represented by the general formula (22), other preferred monomers are those monomers in which R^{22c} is a linear oxyalkylene group having 2 to 20 carbon atoms, preferably 4 to 12 carbon atoms.

Examples of the compounds which correspond to the above preferred monomers and have methacryloyl groups include ethylene glycol dimethacrylate, diethylene glycol dimethacrylate, triethylene glycol dimethacrylate, tetraethylene glycol dimethacrylate, polyethylene glycol dimethacrylate, tripropylene glycol dimethacrylate, tetrapropylene glycol dimethacrylate and polypropylene glycol dimethacrylate.

Examples of the compounds which correspond to the above preferred monomers and have acryloyl groups include ethylene glycol diacrylate, diethylene glycol diacrylate, triethylene glycol diacrylate, tetraethylene glycol diacrylate, polyethylene glycol diacrylate, tripropylene glycol diacrylate, tetrapropylene glycol diacrylate and polypropylene glycol diacrylate.

Among the monomers represented by the general formula (22), other preferred monomers are carbamoyl group-containing monomers represented by the general formula (23) below. 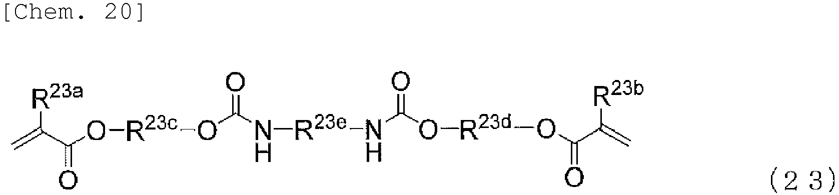 In the general formula (23), R^{23a} and R^{23b} each represent hydrogen or a methyl group and may be the same as or different from each other; and R^{23c} and R^{23a} each represent a C₁₋₁₂ divalent organic group which may contain oxygen, and may be the same as or different from each other.

Examples of the divalent organic groups include hydrocarbon groups, for example, C₁₋₁₂ acyclic hydrocarbon groups such as alkylene groups, and C₁₋₁₂ cyclic hydrocarbon groups such as cycloalkylene groups and arylene groups; and C₁₋₁₂ oxygen-containing hydrocarbon groups such as those groups corresponding to the above hydrocarbon groups except that oxygen is introduced between at least part of the carbon atoms forming carbon-carbon bonds (but oxygen atoms are not inserted contiguously), for example, oxyalkylene groups. The C₁₋₁₂ cyclic hydrocarbon groups may have acyclic hydrocarbon moieties. Further, the acyclic hydrocarbon moieties present in these groups may be linear or branched.

Further, hydrogen atoms present in the organic groups such as the C₁₋₁₂ hydrocarbon groups and the C₁₋₁₂ oxygen-containing hydrocarbon groups may be substituted by acid groups such as carboxyl groups and phosphate groups, functional groups such as hydroxyl groups, amino groups and epoxy groups, and polymerizable groups such as acryloyl groups and methacryloyl groups.

In the general formula (23), R^{23e} represents a C₁₋₂₀ divalent organic group which may contain oxygen.

Examples of the divalent organic groups include hydrocarbon groups, for example, C₁₋₂₀ acyclic hydrocarbon groups such as alkylene groups, and C₁₋₂₀ cyclic hydrocarbon groups such as cycloalkylene groups and arylene groups; and C₁₋₂₀ oxygen-containing hydrocarbon groups such as those groups corresponding to the above hydrocarbon groups except that oxygen is introduced between at least part of the carbon atoms forming carbon-carbon bonds (but oxygen atoms are not inserted contiguously), for example, oxyalkylene groups. The C₁₋₂₀ cyclic hydrocarbon groups may have acyclic hydrocarbon moieties. Further, the acyclic hydrocarbon moieties present in these groups may be linear or branched.

Further, hydrogen atoms present in the organic groups such as the C₁₋₂₀ hydrocarbon groups and the C₁₋₂₀ oxygen-containing hydrocarbon groups may be substituted by acid groups such as carboxyl groups and phosphate groups, and functional groups such as hydroxyl groups, amino groups and epoxy groups.

Examples of the acryloyl group-containing compounds represented by the general formula (23) include urethane acrylates formed by the reaction between a hydroxyacrylate such as 2-hydroxyethyl acrylate, 2-hydroxypropyl acrylate, 2-hydroxybutyl acrylate, 2-hydroxy-3-phenoxypropyl acrylate, 4-hydroxybutyl acrylate or 1,4-cyclohexanedimethanol monoacrylate, and a diisocyanate such as 2,4- or 2,6-toluene diisocyanate, 4,4'-, 2,4'- or 2,2'-diphenylmethane-diisocyanate, 1,6-hexamethylene diisocyanate, or 2,2,4- or 2,4,4-trimethyl-1,6-hexamethylene-diisocyanate. Examples of such urethane acrylates include 2,2,4-trimethylhexamethylene bis(2-carbamoyloxyethyl) diacrylate).

Among the (meth)acryloyl group-containing compounds represented by the general formula (23), other preferred compounds may be at least one selected from the group consisting of compounds represented by the general formulas (24a) to (24e). 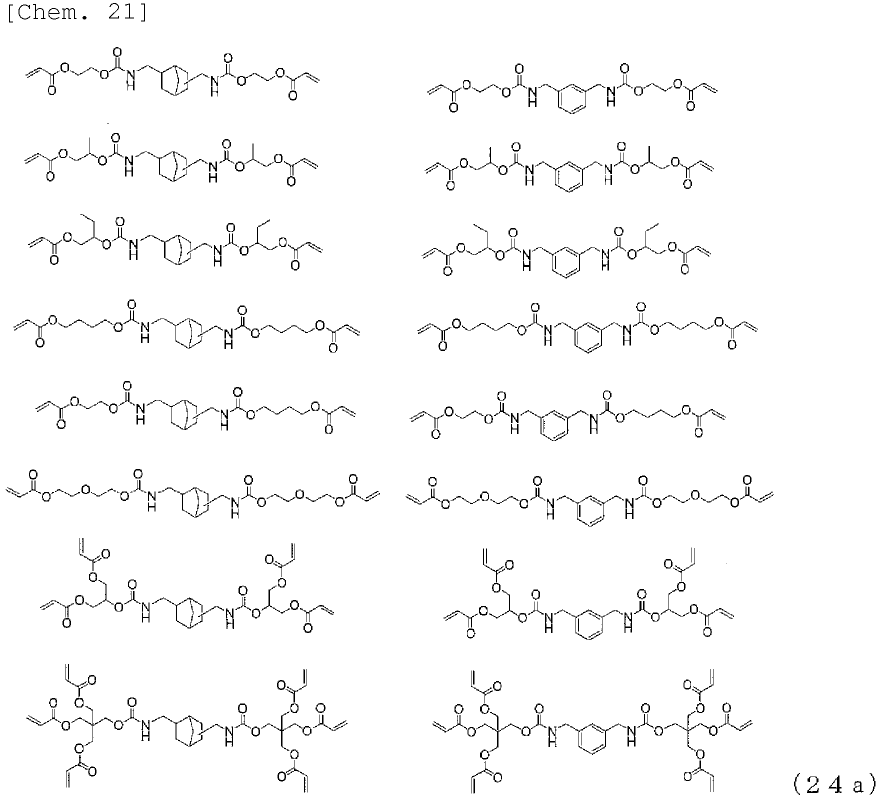 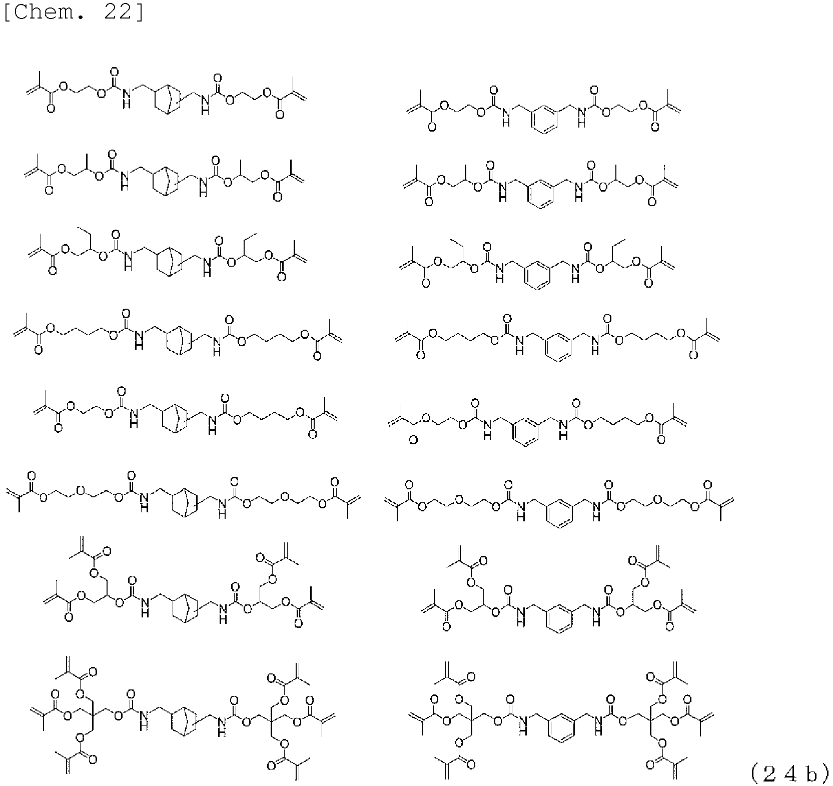 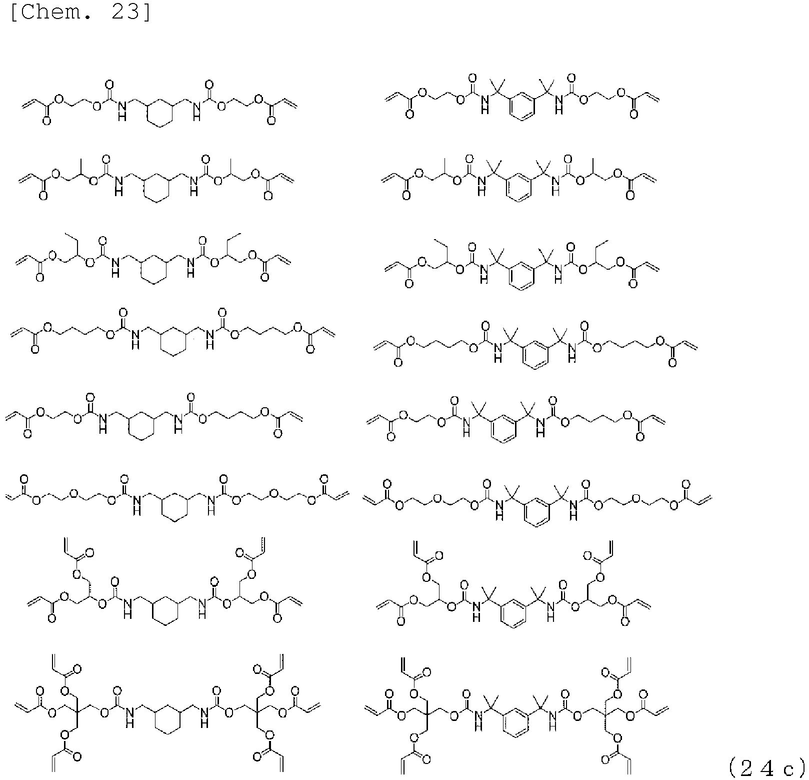 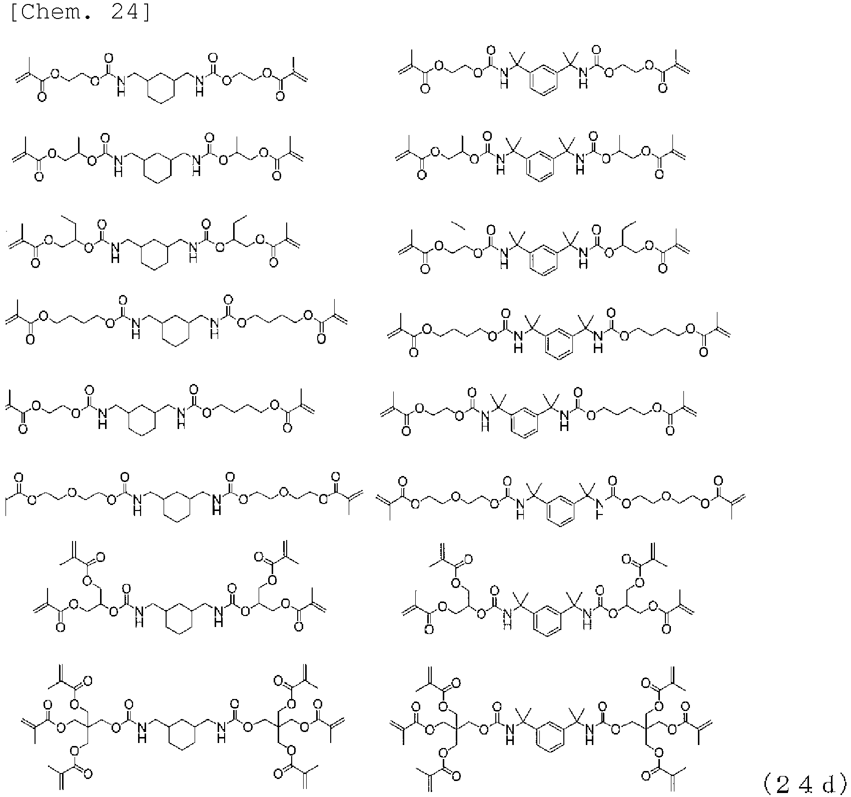 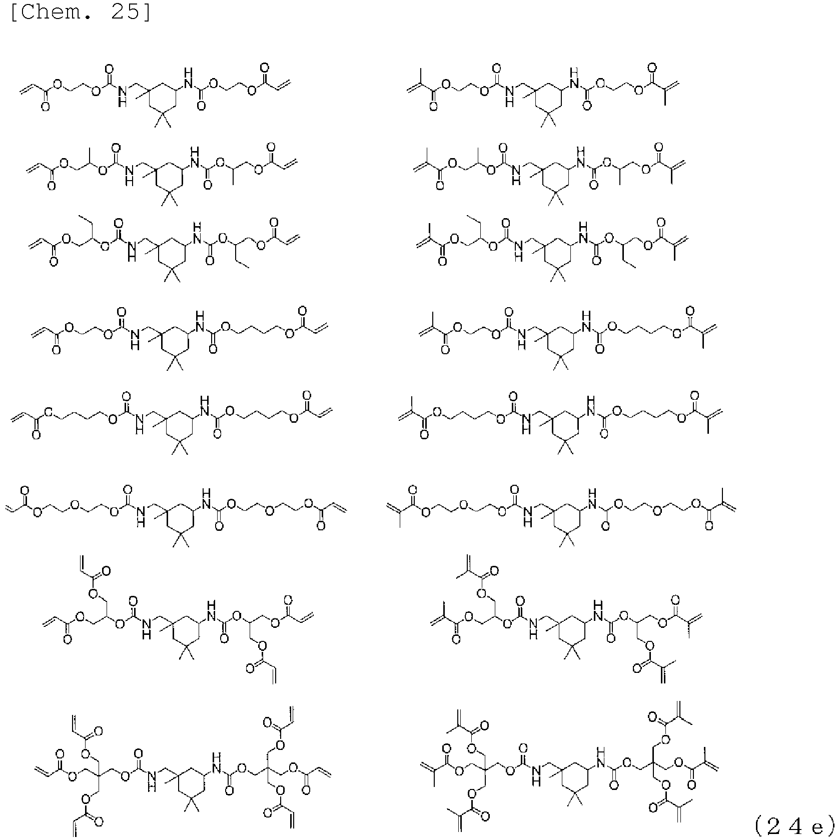 Further, among the monomers represented by the general formula (22), other preferred monomers may be monomers of the general formula (25) below.

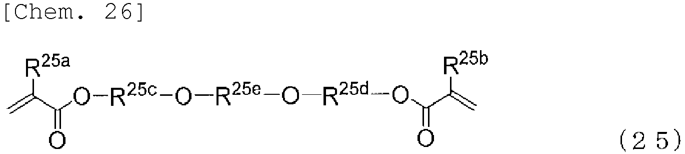

In the general formula (25), R^{25a} and R^{25b} each represent hydrogen or a methyl group and may be the same as or different from each other; and R^{25c} and R^{25d} each represent a C₁₋₁₂ divalent organic group which may contain oxygen, and may be the same as or different from each other. Examples of the divalent organic groups include hydrocarbon groups, for example, C₁₋₁₂ acyclic hydrocarbon groups such as alkylene groups, and C₁₋₁₂ cyclic hydrocarbon groups such as cycloalkylene groups and arylene groups; and C₁₋₁₂ oxygen-containing hydrocarbon groups such as those groups corresponding to the above hydrocarbon groups except that oxygen is introduced between at least part of the carbon atoms forming carbon-carbon bonds (but oxygen atoms are not inserted contiguously), for example, oxyalkylene groups. The C₁₋₁₂ cyclic hydrocarbon groups may have acyclic hydrocarbon moieties. Further, the acyclic hydrocarbon moieties present in these groups may be linear or branched. Further, hydrogen atoms present in the organic groups such as the C₁₋₁₂ hydrocarbon groups and the C₁₋₁₂ oxygen-containing hydrocarbon groups may be substituted by acid groups such as carboxyl groups and phosphate groups, functional groups such as hydroxyl groups, amino groups and epoxy groups, and polymerizable groups such as acryloyl groups and methacryloyl groups.

In the general formula (13), R^{25e} represents a C₁-₂₀ divalent organic group which may contain oxygen. Examples of the divalent organic groups include C₁-₂₀ hydrocarbon groups such as alkylene groups, cycloalkylene groups and arylene groups; and C₁₋₂₀ oxygen-containing hydrocarbon groups such as those groups corresponding to the above hydrocarbon groups except that oxygen is introduced between at least part of the carbon atoms forming carbon-carbon bonds (but oxygen atoms are not inserted contiguously), for example, oxyalkylene groups. The C₁₋₂₀ cyclic hydrocarbon groups may have acyclic hydrocarbon moieties. Further, hydrogen atoms present in the organic groups such as the C₁₋₂₀ hydrocarbon groups and the C₁₋₂₀ oxygen-containing hydrocarbon groups may be substituted by acid groups such as carboxyl groups and phosphate groups, and functional groups such as hydroxyl groups, amino groups and epoxy groups.

Examples of the methacryloyl group-containing compounds represented by the general formula (25) include 2,2-bis[4-(3-methacryloyloxy-2-hydroxypropoxy)phenyl]propane (Bis-GMA), ethylene oxide-modified bisphenol A dimethacrylate and propylene oxide-modified bisphenol A dimethacrylate.

Examples of the acryloyl group-containing compounds represented by the general formula (25) include 2,2-bis[4-(3-acryloyloxy-2-hydroxypropoxy)phenyl]propane, ethylene oxide-modified bisphenol A diacrylate and propylene oxide-modified bisphenol A diacrylate.

When the composition comprising the compound (1) of the invention is used in such an application as adhesive materials, the composition may further contain, as a (meth)acrylate group-containing monomer other than the compounds (1) of the invention, a monomer exhibiting a bonding function. Examples of such adhesive (meth)acrylate group-containing monomers other than the compounds (1) of the invention include monomers having at least one polymerizable group selected from methacryloyl groups and acryloyl groups, and an acidic group (such monomers exclude the compounds (1) of the invention). Examples of the acidic groups include phosphate residues, pyrophosphate residues, thiophosphate residues, carboxylate residues and sulfonate residues.

Examples of the monomers having a methacryloyl group and a phosphate residue (such monomers exclude the compounds (1) of the invention) include 2-methacryloyloxyethyl dihydrogen phosphate, 9-methacryloyloxynonyl dihydrogen phosphate, 10-methacryloyloxydecyl dihydrogen phosphate, 11-methacryloyloxyundecyl dihydrogen phosphate, 20-methacryloyloxyeicosyl dihydrogen phosphate, 1,3-dimethacryloyloxypropyl-2-dihydrogen phosphate, 2-methacryloyloxyethyl phenyl phosphoric acid, 2-methacryloyloxyethyl 2'-bromoethyl phosphoric acid, methacryloyloxyethyl phenyl phosphonate, and acid chlorides of these compounds.

Examples of the monomers having a acryloyl group and a phosphate residue (such monomers exclude the compounds (1) of the invention) include 2-acryloyloxyethyl dihydrogen phosphate, 9-acryloyloxynonyl dihydrogen phosphate, 10-acryloyloxydecyl dihydrogen phosphate, 11-acryloyloxyundecyl dihydrogen phosphate, 20-acryloyloxyeicosyl dihydrogen phosphate, 1,3-diacryloyloxypropyl-2-dihydrogen phosphate, 2-acryloyloxyethyl phenyl phosphoric acid, 2-acryloyloxyethyl 2'-bromoethyl phosphoric acid, acryloyloxyethyl phenyl phosphonate, and acid chlorides of these compounds.

Examples of the monomers having a methacryloyl group and a pyrophosphate residue include di(2-methacryloyloxyethyl) pyrophosphate, and acid chlorides thereof.

Examples of the monomers having an acryloyl group and a pyrophosphate residue include di(2-acryloyloxyethyl) pyrophosphate, and acid chlorides thereof.

Examples of the monomers having a methacryloyl group and a thiophosphate residue include 2-methacryloyloxyethyl dihydrogen dithiophosphate, 10-methacryloyloxydecyl dihydrogen thiophosphate, and acid chlorides of these compounds.

Examples of the monomers having an acryloyl group and a thiophosphate residue include 2-acryloyloxyethyl dihydrogen dithiophosphate, 10-acryloyloxydecyl dihydrogen thiophosphate, and acid chlorides of these compounds.

Examples of the monomers having a methacryloyl group and a carboxylate residue include 4-methacryloyloxyethoxycarbonylphthalic acid, 5-methacryloylaminopentylcarboxylic acid, 11-methacryloyloxy-1,1-undecanedicarboxylic acid, and acid chlorides and acid anhydrides of these compounds.

Examples of the monomers having an acryloyl group and a carboxylate residue include 4-acryloyloxyethoxycarbonylphthalic acid, 5-acryloylaminopentylcarboxylic acid, 11-acryloyloxy-1,1-undecanedicarboxylic acid, and acid chlorides and acid anhydrides of these compounds.

Examples of the monomers having a methacryloyl group and a sulfonate residue include 2-sulfoethyl methacrylate and 2-methacrylamido-2-methylpropanesulfonic acid.

Examples of the monomers having an acryloyl group and a sulfonate residue include 2-sulfoethyl acrylate and 2-acrylamido-2-methylpropanesulfonic acid.

### [Polymerization initiators]

Among the components other than the compounds (1) of the invention in the adhesive materials according to the invention, other components may be polymerization initiators.

The polymerization initiator may be any of general polymerization initiators used in the adhesive materials, and is usually selected in consideration of the polymerizability of the polymerizable monomers, and the polymerization conditions.

In the case of self curing, for example, a redox polymerization initiator that is a combination of an oxidant and a reductant is preferable. When using a redox polymerization initiator, an oxidant and a reductant which are separately packaged need to be mixed with each other immediately before use.

The oxidants are not particularly limited. Examples thereof include organic peroxides such as diacyl peroxides, peroxy esters, dialkyl peroxides, peroxyketals, ketone peroxides and hydroperoxides. Examples of the organic peroxides include such diacyl peroxides as benzoyl peroxide, 2,4-dichlorobenzoyl peroxide and m-toluoyl peroxide; such peroxy esters as t-butyl peroxybenzoate, bis-t-butyl peroxyisophthalate, 2,5-dimethyl-2,5-bis(benzoylperoxy)hexane, t-butyl peroxy-2-ethylhexanoate and t-butyl peroxyisopropyl carbonate; such dialkyl peroxides as dicumyl peroxide, di-t-butyl peroxide and lauroyl peroxide; such peroxyketals as 1,1-bis(t-butylperoxy)-3,3,5-trimethylcyclohexane; such ketone peroxides as methyl ethyl ketone peroxide; and such hydroperoxides as t-butyl hydroperoxide.

The reductants are not particularly limited, but tertiary amines are usually used. Examples of the tertiary amines include N,N-dimethylaniline, N,N-dimethyl-p-toluidine, N,N-dimethyl-m-toluidine, N,N-diethyl-p-toluidine, N,N-dimethyl-3,5-dimethylaniline, N,N-dimethyl-3,4-dimethylaniline, N,N-dimethyl-4-ethylaniline, N,N-dimethyl-4-i-propylaniline, N,N-dimethyl-4-t-butylaniline, N,N-dimethyl-3,5-di-t-butylaniline, N,N-bis(2-hydroxyethyl)-p-toluidine, N,N-bis(2-hydroxyethyl)-3,5-dimethylaniline, N,N-bis(2-hydroxyethyl)-3,4-dimethylaniline, N,N-bis(2-hydroxyethyl)-4-ethylaniline, N,N-bis(2-hydroxyethyl)-4-i-propylaniline, N,N-bis(2-hydroxyethyl)-4-t-butylaniline, N,N-di(2-hydroxyethyl)-3,5-di-i-propylaniline, N,N-bis(2-hydroxyethyl)-3,5-di-t-butylaniline, ethyl 4-dimethylaminobenzoate, n-butoxyethyl 4-dimethylaminobenzoate, (2-methacryloyloxy)ethyl 4-dimethylaminobenzoate, trimethylamine, triethylamine, N-methyldiethanolamine, N-ethyldiethanolamine, N-n-butyldiethanolamine, N-lauryldiethanolamine, triethanolamine, (2-dimethylamino)ethyl methacrylate, N,N-bis(methacryloyloxyethyl)-N-methylamine, N,N-bis(methacryloyloxyethyl)-N-ethylamine, N,N-bis(2-hydroxyethyl)-N-methacryloyloxyethylamine, N,N-bis(methacryloyloxyethyl)-N-(2-hydroxyethyl)amine and tris(methacryloyloxyethyl)amine.

Besides these organic peroxide/amine systems, other redox polymerization initiators such as cumene hydroperoxide/thiourea systems, ascorbic acid/Cu²⁺ salt systems and organic peroxide/amine/sulfinic acid (or sulfinate salt) systems may be used. Further, other polymerization initiators such as tributyl borane and organic sulfinic acids are also suitably used.

In the case of thermal polymerization with heating, it is preferable to use peroxides or azo compounds.

The peroxides are not particularly limited, and examples thereof include benzoyl peroxide, t-butyl hydroperoxide and cumene hydroperoxide. The azo compounds are not particularly limited, and examples thereof include azobisisobutyronitrile.

In the case of photopolymerization with the application of visible lights, suitable initiators are redox initiators such as α-diketones/tertiary amines, α-diketones/aldehydes and α-diketones/mercaptans.

Examples of the photopolymerization initiators, although not particularly limited to, include α-diketones/reductants, ketals/reductants and thioxanthones/reductants. Examples of the α-diketones include camphorquinone, benzil and 2,3-pentanedione. Examples of the ketals include benzyl dimethyl ketal and benzyl diethyl ketal. Examples of the thioxanthones include 2-chlorothioxanthone and 2,4-diethylthioxanthone. Examples of the reductants include tertiary amines such as Michler's ketone, 2-(dimethylamino)ethyl methacrylate, N,N-bis[(meth)acryloyloxyethyl]-N-methylamine, ethyl N,N-dimethylaminobenzoate, butyl 4-dimethylaminobenzoate, butoxyethyl 4-dimethylaminobenzoate, N-methyldiethanolamine, 4-dimethylaminobenzophenone, N,N-bis(2-hydroxyethyl)-p-toluidine and dimethylaminophenanthrol; aldehydes such as citronellal, lauryl aldehyde, phthalic dialdehyde, dimethylaminobenzaldehyde and terephthalaldehyde; and thiol group-containing compounds such as 2-mercaptobenzoxazole, decanethiol, 3-mercaptopropyltrimethoxysilane, 4-mercaptoacetophenone, thiosalicylic acid and thiobenzoic acid. Organic peroxides may be added to these redox systems. That is, α-diketone/organic peroxide/reductant systems may be suitably used.

In the case of photopolymerization with the application of UV lights, some suitable initiators are benzoin alkyl ethers and benzyl dimethyl ketal. Further, such photopolymerization initiators as (bis)acylphosphine oxides are also suitably used.

Of the (bis)acylphosphine oxides, examples of the acylphosphine oxides include 2,4,6-trimethylbenzoyldiphenylphosphine oxide, 2,6-dimethoxybenzoyldiphenylphosphine oxide, 2,6-dichlorobenzoyldiphenylphosphine oxide, 2,4,6-trimethylbenzoylmethoxyphenylphosphine oxide, 2,4,6-trimethylbenzoylethoxyphenylphosphine oxide, 2,3,5,6-tetramethylbenzoyldiphenylphosphine oxide and benzoyldi-(2,6-dimethylphenyl) phosphonate. Examples of the bisacylphosphine oxides include bis-(2,6-dichlorobenzoyl)phenylphosphine oxide, bis-(2,6-dichlorobenzoyl)-2,5-dimethylphenylphosphine oxide, bis-(2,6-dichlorobenzoyl)-4-propylphenylphosphine oxide, bis-(2,6-dichlorobenzoyl)-1-naphthylphosphine oxide, bis-(2,6-dimethoxybenzoyl)phenylphosphine oxide, bis-(2,6-dimethoxybenzoyl)-2,4,4-trimethylpentylphosphine oxide, bis-(2,6-dimethoxybenzoyl)-2,5-dimethylphenylphosphine oxide, bis-(2,4,6-trimethylbenzoyl)phenylphosphine oxide and (2,5,6-trimethylbenzoyl)-2,4,4-trimethylpentylphosphine oxide. These (bis)acylphosphine oxide photopolymerization initiators may be used singly or in combination with various reductants such as amines, aldehydes, mercaptans and sulfinate salts. These reductants may be suitably used also in combination with the visible light photopolymerization initiators described hereinabove.

The polymerization initiators or the photopolymerization initiators may be used singly, or two or more thereof may be used in appropriate combination. The amount thereof is usually in the range of 0.01 to 20 parts by weight, preferably 0.1 to 5 parts by weight per 100 parts by weight of the dental material.

### [Fillers]

Among the components other than the compounds (1) of the invention in the adhesive materials according to the invention, other components may be fillers.

The filler may be any of general fillers used in the adhesive material field. The fillers are usually broadly categorized into organic fillers and inorganic fillers.

Examples of the organic fillers include fine powders of polymethyl methacrylate, polyethyl methacrylate, methyl methacrylate-ethyl methacrylate copolymer, crosslinked polymethyl methacrylate, crosslinked polyethyl methacrylate, ethylene-vinyl acetate copolymer and styrene-butadiene copolymer.

Examples of the inorganic fillers include fine powders of various glasses (based on silicon dioxide and optionally containing oxides of, for example, heavy metals, boron and aluminum), various ceramics, diatomaceous earth, kaolin, clay minerals (such as montmorillonite), activated clay, synthetic zeolite, mica, calcium fluoride, ytterbium fluoride, calcium phosphate, barium sulfate, zirconium dioxide, titanium dioxide and hydroxyapatite. As specific examples of such inorganic fillers, those that are used as X-ray contrast agents are preferable. Examples of the X-ray contrast agents include barium borosilicate glasses (such as Kimble Raysorb T3000, Schott 8235, Schott GM27884 and Schott GM39923), strontium boroaluminosilicate glasses (such as Raysorb T4000, Schott G018-093 and Schott GM32087), lanthanum glasses (such as Schott GM31684), fluoroaluminosilicate glasses (such as Schott G018-091 and Schott G018-117), and boroaluminosilicate glasses containing zirconium and/or cesium (such as Schott G018-307, G018-308 and G018-310).

Further, an organic inorganic composite filler may be used which is obtained by adding a polymerizable monomer beforehand to the inorganic filler to give a paste, which is then cured by polymerization and crushed.

In a preferred aspect of the adhesive material, the material contains a microfiller having a particle diameter of not more than 0.1 um. Such a material is, for example, suited as a dental composite resin. Preferred examples of the materials for such micron size fillers include silica (for example, product name: AEROSIL), alumina, zirconia and titania. The addition of such a micron size inorganic filler is advantageous in order for a cured product of the composite resin to achieve high polish and smoothness by being polished.

These fillers may have been surface treated with agents such as silane coupling agents in accordance with purposes. Examples of such surface treating agents include known silane coupling agents, for example, organosilicon compounds such as γ-methacryloxyalkyltrimethoxysilanes (the number of carbon atoms between the methacryloxy group and the silicon atom: 3 to 12), γ-methacryloxyalkyltriethoxysilanes (the number of carbon atoms between the methacryloxy group and the silicon atom: 3 to 12), vinyltrimethoxysilane, vinylethoxysilane and vinyltriacetoxysilane. The surface treating agent is usually used with a concentration in the range of 0.1 to 20 parts by weight, and preferably 1 to 10 parts by weight per 100 parts by weight of the filler.

These fillers may be appropriately added according to the purpose of the adhesive material. These fillers are appropriately used singly, or two or more thereof are appropriately used in combination. The preferred range of the amount of the filler varies depending on the purpose of the dental material. For example, in the case where the adhesive material is used as a dental bonding material which is one of adhesive materials, the filler may be added in an amount of about 0.1 to 5 parts by weight per 100 parts by weight of the dental material in order to adjust the viscosity of the dental material. As another example, in the case where the dental material is used as a dental adhesive cement which is one of dental adhesives, the filler may be added in an amount of about 30 to 70 parts by weight per 100 parts by weight of the dental material in order to enhance the mechanical strength and adjust the viscosity. As still another example, in the case where the dental material is used as a dental adhesive composite resin which is one of dental adhesives, the filler may be added in an amount of about 50 to 90 parts by weight per 100 parts by weight of the dental material in order to enhance the mechanical strength and adjust the viscosity.

### [Other components]

The adhesive material according to the invention may appropriately contain components other than the compounds (1) of the invention, the polymerizable monomer other than the compounds (1) of the invention (for example the (meth)acrylate group-containing monomer other than the compounds (1) of the invention, or the epoxy group-containing monomer), the polymerization initiator and the filler in accordance with the purpose. For example, the dental material may contain the aforementioned polymerization inhibitor for enhancing storage stability. To control the color tone, known colorants such as pigments and dyes may be added. To enhance the strength of the cured product, known reinforcing materials such as fibers may further be added. In addition, solvents such as acetone, ethanol, water, ethyl acetate and toluene may be added as necessary.

### [Ratio of components]

The amount of the compound (1) of the invention based on the amount of the adhesive material is not particularly limited, and is, for example, in the range of 0.1 to 99%. The preferred amount of the compound (1) may vary depending on the purpose of the adhesive material. For example, the compound (1) is added in an amount of 1 to 60 wt% based on the amount of the polymerizable monomer components (the compound (1) of the invention and the polymerizable monomer other than the compounds (1) of the invention (for example the (meth)acrylate group-containing monomer other than the compounds (1) of the invention, or the epoxy group-containing monomer)). In particular, when the dental material is used as a bonding material, an adhesive resin cement, a filling adhesive composite resin or a dental fissure sealant, the amount of the compound (1) of the invention is preferably 1 to 50 wt%, more preferably 3 to 30 wt% based on the amount of polymerizable monomer components.

### [Reverse mutation test]

The dental material according to the invention is preferably negative in a reverse mutation test. The reverse mutation test (Ames test) is conducted in accordance with the same procedure as that of the above-described reverse mutation test except that the dental material according to the invention is used in place of the composition according to the invention.

### [Cytotoxicity test in NRU method]

The dental material according to the invention may allow the relative cell survival rate to fall within a certain range in a cytotoxicity test in the NRU method using Balb/3T3 cells. The cytotoxicity test is conducted in accordance with the same procedure as that of the above-described cytotoxicity test in the NRU method except that the composition according to the invention is used in place of the dental material according to the invention. Further, the concentration of the test substance of the test material (the compound (1) of the dental material) in the test solution, and the relative cell growth rate (%) in the dental material may be the same as in the case of the composition according to the invention.

### [Cytotoxicity test in WST method]

The dental material according to the invention may allow the relative cell growth rate to fall within a certain range in a cytotoxicity test in the WST method using Balb/3T3 cells. The cytotoxicity test is conducted in accordance with the same procedure as that of the above-described cytotoxicity test in the WST method except that the composition according to the invention is used in place of the dental material according to the invention. Further, the concentration of the test substance of the test material (the compound (1) of the dental material) in the test solution, and the relative cell growth rate (%) in the dental material may be the same as in the case of the composition according to the invention.

### [Method for producing adhesive material]

A known method may be adopted without limitation as a method for producing the adhesive material according to the invention by mixing the compound (1) of the invention, the polymerizable monomer other than the compound (1) of the invention (for example the (meth)acrylate group-containing monomer other than the compounds (1) of the invention, or the epoxy group-containing monomer), the polymerization initiator, the filler, and other components

### [Cured product]

The adhesive material according to the invention may be cured under appropriate conditions in accordance with the manner in which the polymerization initiator initiates the polymerization. In the case where, for example, the adhesive material according to the invention contains a visible light photopolymerization initiator, a desired cured product may be obtained by shaping the adhesive material into a prescribed form, and then irradiating the material with visible light for a prescribed time using a known irradiator. The conditions such as intensity and dose may be controlled appropriately in accordance with the curability of the adhesive material. The cured product that has been cured by the light irradiation such as visible light may be heat treated under more appropriate conditions, and thereby the mechanical properties of the cured product can be enhanced. Alternatively, in the case where the dental material according to the invention contains a heat polymerization initiator, a desired cured product may be obtained by shaping the dental material into a prescribed form, and then heating the material at an appropriate temperature for an appropriate time.

The thus-obtained cured product of the adhesive material according to the invention may be used for various purposes such as dental treatment purposes.

### [Purposes]

The adhesive material according to the present invention may be used for purposes in which adhesiveness is required. For example, the adhesive material may be used for bonding the same type of films or different types of films to each other, or bonding the same type of structures or different types of structures to each other, or may be used for coating the surfaces of certain materials. Further, the adhesive material may be combined with other adhesive materials, and used as primers for adherends.

Any adherends on which the inventive compounds (1) having phosphor-containing groups exhibit an adhesivenessrelated effect may be used without limitation, and examples of the preferred adherends may include metallic materials with which phosphate groups are expected to strongly interact, ceramic materials, and (meth)acrylate-based materials which are expected to be copolymerized with (meth)acryloyl groups.

The adhesive material according to the invention may also be used as a dental material. Examples thereof may include a orthodontic adhesive material, a bonding material, an adhesive resin cement, a filling adhesive composite resin, a dental fissure sealant and a resin glass ionomer cement.

While details of the reason why the compound (1) of the invention may be particularly suitably used for adhesive materials are unknown, the compound (1) of the invention contains both phosphate groups and (meth)acryloyl groups in the molecule as described above, and is thus supposed to have the three functions: a metal surface etching ability derived from phosphoric acid as acidic groups, interaction with the metal surface via phosphate groups and bonding to a resin matrix via (meth)acryloyl groups. Further, the phosphorus-containing compound (1) of the invention has a carbamate structure in the molecule. The carbamate structure is known to exhibit the effect of imparting high mechanical properties to a so-called urethane polymer due to the coagulation effect of the carbamate structure in the urethane polymer, and in the invention, the carbamate structure present in the molecule of the compound (1) of the invention may also have a favorable effect on the strength of the cured adhesive material according to the invention.

### [Usage]

The adhesive material of the invention may be used by any known methods generally adopted for adhesive materials without limitation. When, for example, the adhesive material according to the invention is used as, for example, a dental bonding material, the adhesive material is applied to a cavity in the mouth, then dried as necessary, and photocured with a known irradiator as necessary, and a filling composite resin is then packed.

When, for example, the adhesive material of the invention is used as a dental adhesive resin cement, a tooth surface and a prosthesis bonded surface are treated with a primer as necessary, the adhesive material of the invention is then applied to the prosthesis, and the prosthesis is pressure-bonded at a prescribed site in the mouth.

When, for example, the adhesive material of the invention is used as a tooth primer, the adhesive material is applied to a cavity in the mouth, then dried as necessary, and photocured with a known irradiator as necessary, and a prosthesis coated with an adhesive cement is pressure-bonded to the cavity.

When, for example, the adhesive material according to the invention is used as a filling adhesive composite resin, the dental material is directly packed in a cavity in the mouth, and then photocured with a known irradiator to achieve the purpose.

### [Kits]

Kits according to the invention include the adhesive material. The kits according to the invention include kits in which each component of the adhesive material is packed as one agent; and kits composed of a plurality of agents such that each component of the dental material is divided into two or more agents and packed in view of a polymerization type, and storage stability. The kits according to the invention may include other adhesive materials which are used in combination with the adhesive material according to the invention. Such kits are used for various purposes such as dental purposes.

### EXAMPLES

The present invention will be described in further detail based on Examples hereinbelow without limiting the scope of the invention to such Examples.

### [Production Example 1]

A 500-milliliter four-necked flask equipped with a stirring blade, a thermometer and a reflux tube was loaded with 100 g (1.09 mol, the number of moles of OH groups: 3.27 mol) of glycerin (manufactured by Sigma-Aldrich Co. LLC), 0.43 g (1000 ppm based on the total weight of reactants) of dibutyltin dilaurate (manufactured by Wako Pure Chemical Industries, Ltd.) and 0.22 g (500 ppm based on the total weight of reactants) of 2,6-t-butyl-4-methylphenol (manufactured by Wako Pure Chemical Industries, Ltd.), and heated to 55°C. Subsequently, 337 g (2.17 mol, 2/3 equivalents based on the number of moles of OH groups of glycerin used) of 2-methacryloyloxyethyl isocyanate (KARENZ MOI (registered trademark), manufactured by Showa Denko K.K.) was added dropwise over a period of 30 minutes. Reaction was carried out for 4 hours at a temperature of 80 to 85°C. The infrared absorption spectrum IR was measured (Spectrum Two, manufactured by PerkinElmer), and the result showed that the isocyanate-derived vibration at 2267 cm⁻¹ disappeared. A part of the product was taken, the hydroxyl group value thereof was measured in accordance with JIS K 0070, and the result showed that the hydroxyl group value was 138 mg KOH/g. The reaction product was subjected to liquid chromatography mass spectrometry (LC-MS analysis) (1.7 um ACQUITY UPLC BEH C18 (2.1 mm × 10 mm)/ACQUITY UPLC H-Class-SQ Detector 2, manufactured by Nihon Waters K.K.), and the result showed that the mass of [M-H]⁺ was 403. This result demonstrated that the reaction product had a molecular weight of 402, and this molecular weight was consistent with that of the compound 1. The reaction product was discharged from the reaction vessel to give 417 g of a product containing the compound 1 below.

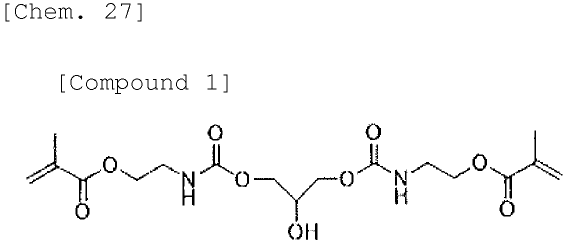

Subsequently, a 300-milliliter four-necked flask equipped with a stirring blade, a thermometer and a reflux tube was loaded with 152 g (hydroxyl group value: 138 mg KOH/g, the number of moles of OH groups: 0.374 mol) of the reaction product containing the compound 1, 450 mL of ultradehydrated methylene chloride (manufactured by Wako Pure Chemical Industries, Ltd.) and 0.093 g (500 ppm based on the total weight of reactants) of 2,6-t-butyl-4-methylphenol. Subsequently, 34.1 g (0.240 mol, P equivalent: 0.480 mol) of diphosphorus pentaoxide (manufactured by Tokyo Chemical Industry Co., Ltd.) was added in three separate portions. Reaction was carried out for 6 hours at a reaction temperature equal to or higher than room temperature and not higher than 30°C. Thereafter, 150 mL of water was slowly added into the apparatus to completely deactivate unreacted diphosphorus pentaoxide at room temperature. The organic layer was extracted, and volatile components were distilled off. The reaction product was discharged from the reaction vessel to give 150 g of a product containing a phosphoric acid ester-containing urethane methacrylic compound of the structural formulas of the compounds 2-1 and 2-2 below. The reaction product was subjected to LC-MS analysis, and the result showed that the masses of [M-H]⁺ were 483 and 867. This result indicated that the main products had a molecular weight of 482 and a molecular weight of 866, which were consistent with the molecular weight of the compound 2-1 below and the molecular weight of the compound 2-2 below, respectively.

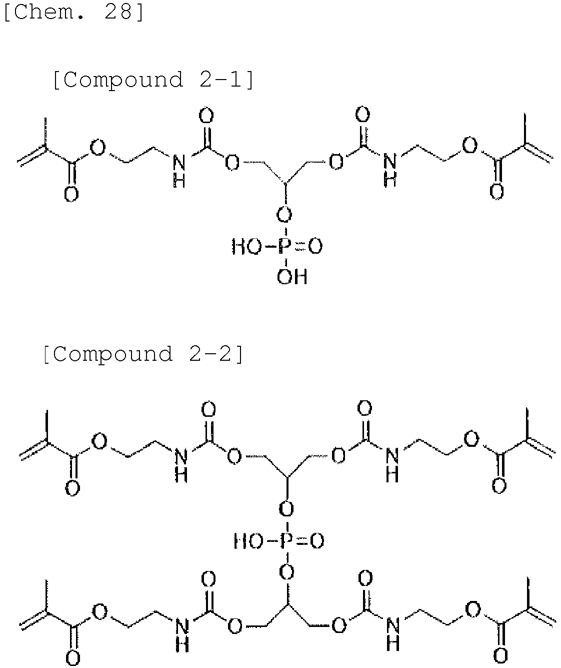

### [Production Example 2]

Except that diglycerin (manufactured by NACALAI TESQUE, INC.) was used in place of the glycerin described in Production Example 1, the same synthesis operation as in Production Example 1 was carried out to give a product containing a compound 3 of the structural formula of the compound 3 below, and a phosphoric acid ester-containing compound 4 of the structural formula of the compound 4 below.

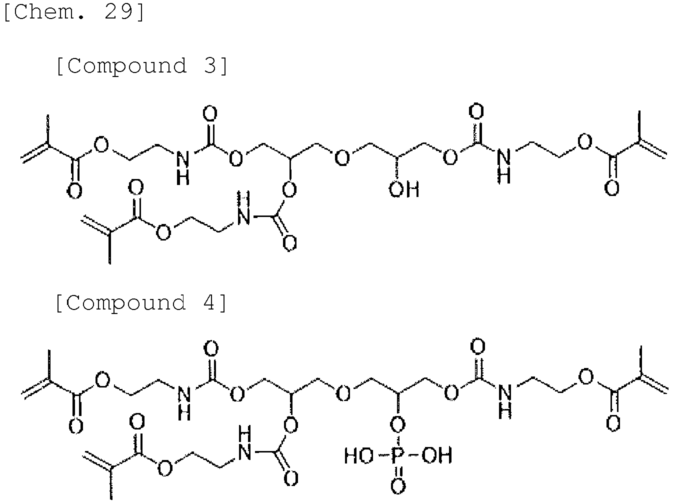

### [Production Example 3]

Except that KARENZ MOI-EG (registered trademark) (manufactured by Showa Denko K.K.) containing ethylene glycol units in the molecule was used in place of the 2-methacryloyloxyethyl isocyanate (KARENZ MOI (registered trademark)) described in Production Example 1, the same synthesis operation as in Production Example 1 was carried out to give a product containing a compound 5 of the structural formula of the compound 5 below, and a phosphoric acid ester-containing compound 6 of the structural formula of the compound 6 below.

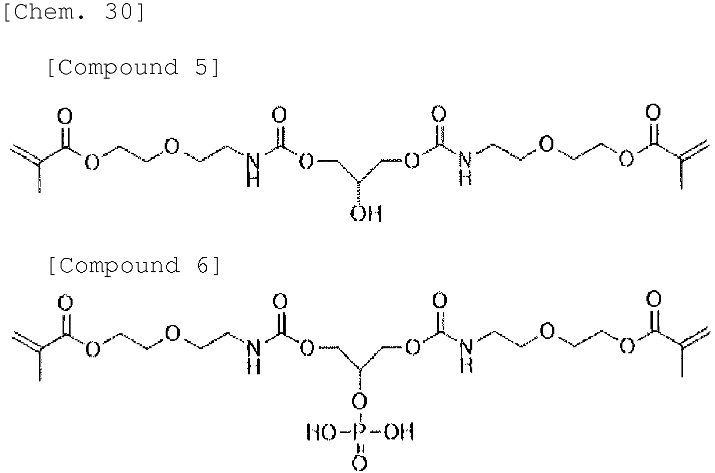

### [Table 1]

**Table 1**

| Production Examples | Structural formulas of alcohol raw materials | Structural formulas of monophosphoric acid ester group-containing compound precursors/hydroxyl values (meKOH/g) | Monophosphoric acid ester group-containing compounds |
|---|---|---|---|
| 1 | 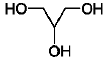 | 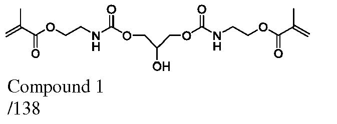 | 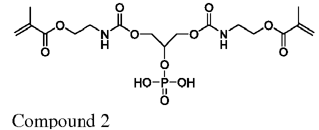 |
| 2 | 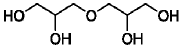 | 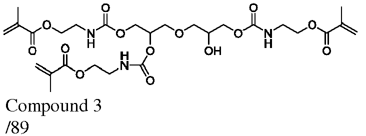 | 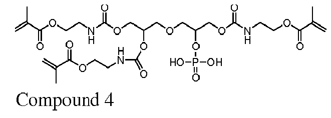 |
| 3 | 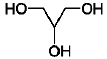 | 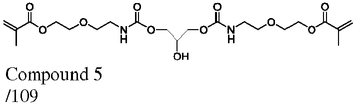 | 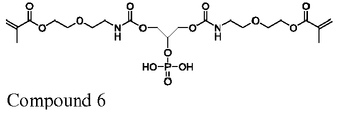 |

### [Example 1]

0.80 g (1.7 mmol) of the compound 2 obtained in Production Example 1, 2.5 g (5.3 mmol) of UDMA (2,2,4-trimethylhexamethylenebis(2-carbamoyloxyethyl) dimethacrylate) and 0.74 g (2.6 mmol) of TEGDMA (triethylene glycol dimethacrylate: NK Ester 3G, manufactured by Shin-Nakamura Chemical Co, Ltd.) were added into a container, and stirred to uniformity at 50°C to give a polymerizable monomer composition. 0.5 parts by weight of TPO (2,4,6-trimethylbenzoyl-diphenyl-phosphine oxide: IRGACURE TPO, manufactured by BASF SE) was then added to and mixed with 100 parts by weight of the polymerizable monomer composition to give a uniform pasty composition to be used as a dental material. The composition of Example 1 is an example of compositions suitable for evaluation of performance as a resin.

### [Bending strength test method]

The composition prepared as described above was packed in a 2 × 2 × 25 mm SUS mold, covered with a cover film, and then irradiated with light from a dental visible light irradiator (α Light V, manufactured by J. Morita Tokyo MFG. Corp.) for 3 minutes on each side, namely, for a total of 6 minutes on both sides to cure the composition. The cured product was stored in deionized water at 37°C for 24 hours, and then subjected to a three-point bending test with a general-purpose tester (Precise Versatile Material Tester 210X, manufactured by INTESCO Co., Ltd.) under conditions in which the distance between supports was 20 mm and the cross head speed was 1.0 mm/min. The results of the bending test of the cured products of the compositions to be used as the dental materials are shown in Table 2.

### [Adhesive strength test method]

A bovine lower anterior tooth extracted and kept in a frozen state was thawed by injection of water, and subjected to root amputation and pulp extirpation treatment. This was placed in a plastic cylindrical container having a diameter of 25 mm and a depth of 25 mm, and embedded in an acrylic resin. The surface thereof was wet-polished with #120 and #400 emery papers to expose enamel in a state of being parallel to the lip surface.

Next, compressed air was blown onto the flat surface for about 1 second to perform drying, the prepared composition was then applied to the enamel flat surface, and compressed air was blown with a low blowing force. This surface was irradiated with light from a visible light irradiator (Translux 2Wave, manufactured by Heraeus Kulzer GmbH) for 20 seconds. Further, a plastic mold having a diameter of 2.38 mm (manufactured by Ultradent Products, Inc.) was then placed thereon, and a dental composite resin (Venus Diamond, manufactured by Heraeus Kulzer GmbH) was packed, irradiated with light from the visible light irradiator for 20 seconds, and thereby cured. Thereafter, the mold was removed to prepare an adhesive sample. The sample was stored in warm water at 37°C for 24 hours, and a shear load which was parallel to the enamel and in contact with the surface of the bovine tooth was then applied at a cross head speed of 1.0 mm/min using a general-purpose tester (Precise Versatile Material Tester 210X, manufactured by INTESCO Co., Ltd.). The shear adhesive strength was determined from the shear load at the time when the columnar composition formed on the bovine tooth surface was separated from the surface.

The results of the shear tests of the compositions for dental materials are shown in Table 2.

### [Examples 2 and 3]

Except that the phosphoric acid ester group-containing methacrylic compounds obtained in Production Examples 2 and 3 were used in place of the compound 2, the same operation as in Example 1 was carried out to prepare polymerizable monomer compositions, and compositions to be used as dental materials. Subsequently, the same tests as in Example 1 were conducted to obtain bending strength and shear test results. The results are shown in Table 2.

### [Comparative Example 1]

Except that MDP (10-methacryloyloxydecyl dihydrogen phosphate) was used in place of the compound 2, the same operation as in Example 1 was carried out to prepare a polymerizable monomer composition, and a composition to be used as a dental material. Subsequently, the same tests as in Example 1 were conducted to obtain bending strength and shear test results. The results are shown in Table 2.

### [Table 2]

**Table 2**

| | Phosphoric acid ester group-containing methacrylic compounds | Results of shear test on bovine tooth (MPa) | Cured product bending test results | |
|---|---|---|---|---|
| | | | Maximum stress (MPa) | Elastic modulus (GPa) |
| Example 1 | Compound 2 | 17.7± 3.4 | 102 | 2.3 |
| Example 2 | Compound 4 | 18.1± 4.9 | 102 | 2.4 |
| Example 3 | Compound 6 | 15.2± 5.4 | 89 | 2.0 |
| Comparative Example 1 | MDP | 14.5± 2.4 | 85 | 1.7 |

The results in Table 2 reveal that the compositions containing phosphorus-containing compounds of the invention have higher adhesive strength as compared to the composition of the conventional adhesive monomer. Thus, use of the phosphorus-containing compound of the invention enhances the adhesive strength of adhesive materials.

### [Example 4]

0.48 g (8.0 parts by weight) of the compound 2 obtained in Production Example 1, 1.2 g (20 parts by weight) of UDMA (2,2,4-trimethylhexamethylenebis(2-carbamoyloxyethyl) dimethacrylate), 0.6 g (10 parts by weight) of TEGDMA (triethylene glycol dimethacrylate: NK Ester 3G, manufactured by Shin-Nakamura Chemical Co, Ltd.), 0.12 g (2.0 parts by weight) of 4-META (4-methacryloyloxyethyl trimellitic anhydride, manufactured by Wako Pure Chemical Industries, Ltd.), 0.012 g (0.2 parts by weight) of CQ (camphaquinone, manufactured by Wako Pure Chemical Industries, Ltd.) and 0.024 g (0.4 parts by weight) of 2-butoxyethyl 4-(dimethylamino)benzoate (manufactured by Tokyo Chemical Industry Co., Ltd.) were added into a container, and stirred to uniformity at 50°C to give a mixture. To the mixture was added 3.6 g (59 parts by weight) of a barium aluminum borosilicate glass filler (GM 27884, particle diameter: 1.5 um, 1.6% silane-treated product, manufactured by NEC SCHOTT Components Corporation), and the resulting mixture was mixed to give a uniform pasty composition to be used as a dental material. The composition of Example 4 is an example of compositions suitable for evaluation of performance as a resin. Except that bovine tooth dentin was used, a plastic mold was placed on a dentin flat surface blown with compressed air, the composition was packed in two portions, and a dental composite resin (Venus Diamond) was not used, the same tests as in Example 1 were conducted to obtain bending test and shear test results. The results are shown in Table 3.

### [Comparative Example 2]

Except that MDP (10-methacryloyloxydecyl dihydrogen phosphate) was used in place of the compound 2, the same operation as in Example 4 was carried out to prepare a composition to be used as a dental material. Subsequently, the same tests as in Example 4 were conducted to obtain bending strength and shear test results. The results are shown in Table 3.

### [Table 3]

**Table 3**

| | Phosphoric acid ester group-containing methacrylic compounds | Results of shear test on bovine tooth | Cured product bending test results |
|---|---|---|---|
| | | Dentin (MPa) | Elastic modulus (GPa) |
| Example 4 | Compound 2 | 20.2±4.0 | 6.5 |
| Comparative Example 2 | MDP | 10.4±2.8 | 5.7 |

### [Example 5]

1.0 g (4.7 parts by weight) of the compound 2 obtained in Production Example 1, 6.0 g (28 parts by weight) of Bis-GMA (bisphenol A diglycidyl methacrylate, manufactured by Shin-Nakamura Chemical Co, Ltd.), 6.0 g (28 parts by weight) of HEMA (Acryester HO (registered trademark), manufactured by Mitsubishi Rayon Co., Ltd.), 0.020 g (0.94 parts by weight) of TPO (2,4,6-trimethylbenzoyl-diphenyl-phosphine oxide: IRGACURE TPO, manufactured by BASF SE), 0.40 g (1.9 parts by weight) of CQ (camphaquinone, manufactured by Wako Pure Chemical Industries, Ltd.), 0.20 g (0.94 parts by weight) of ethyl 4-(dimethylamino)benzoate (manufactured by Wako Pure Chemical Industries, Ltd.), 0.20 g (0.94 parts by weight) of p-tolyldiethanolamine (manufactured by Tokyo Chemical Industry Co., Ltd.), 3.0 g (14 parts by weight) of BHT (dibutylhydroxytoluene: manufactured by Wako Pure Chemical Industries, Ltd.), 3.0 g of ethanol (ultradehydrated: manufactured by Wako Pure Chemical Industries, Ltd.) and 3.0 g (14 parts by weight) of distilled water were added into a container, and stirred to uniformity at 50°C to give a mixture. To the mixture was added 1.2 g (5.7 parts by weight) of a barium aluminum borosilicate glass filler (GM 27884, particle diameter: 1.5 um, 1.6% silane-treated product, manufactured by NEC SCHOTT Components Corporation), and the resulting mixture was mixed to give a uniform liquid composition to be used as a dental material. The composition of Example 5 is an example of compositions suitable for evaluation of performance as a resin. Except that bovine tooth dentin was used, and after application of the composition, compressed air was blown with a low blowing force to remove the solvent, the same test as in Example 1 was conducted to obtain a shear test result. The result is shown in Table 4.

### [Comparative Example 3]

Except that MDP (10-methacryloyloxydecyl dihydrogen phosphate) was used in place of the compound 2, the same operation as in Example 5 was carried out to prepare a composition to be used as a dental material. Subsequently, the same test as in Example 5 was conducted to obtain a shear test result. The result is shown in Table 4.

### [Table 4]

**Table 4**

| | Phosphoric acid ester group-containing methacrylic compounds | Results of shear test on bovine tooth |
|---|---|---|
| | | Dentin (MPa) |
| Example 5 | Compound 2 | 28.5±4.9 |
| Comparative Example 3 | MDP | 19.4±5.8 |

The results in Tables 3 and 4 have shown that the composition of the phosphoric acid ester group-containing methacrylic compound of the invention has higher adhesive strength to the tooth as compared to the composition of the conventional phosphoric acid ester group-containing methacrylic compound.

## Claims

1. A compound represented by the general formula (1) below, in which the core (X) below and the terminal group (Y1) below are bonded to each other:
[Chem. 1]
X (Y1) n¹ (1)
wherein in the general formula (1), n¹ represents the number of terminal groups (Y1) bonded to the core (X), and n¹ is equal to the valence of the core (X); and
the terminal group (Y1) is a phosphorus-containing group represented by the general formula (2) below, a phosphorus-containing group represented by the general formula (3) below, a (meth)acryloyl group-containing group (Y2) represented by the general formula (4) below, a (meth) acryloyl group, a C₁₋₂₀ hydrocarbon group or a hydrogen atom, and a plurality of terminal groups (Y1) may be the same as or different from each other, with the proviso that among all the terminal groups (Y1) in the compound represented by the general formula (1), one or more terminal groups are phosphorus-containing groups represented by the general formula (2) below or phosphorus-containing groups represented by the general formula (3) below, and one or more terminal groups are (meth)acryloyl group-containing groups (Y2); 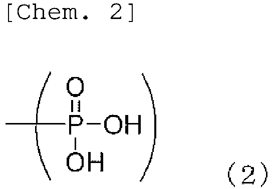 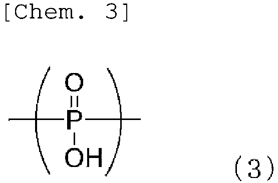
wherein in the general formula (3), one end of the group is bonded to the core (X), and the other end of the group is bonded to the core (X) present in another compound represented by the general formula (1); and 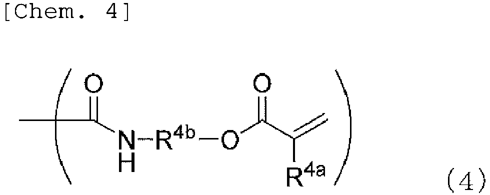
wherein in the general formula (4), R^{4a} represents a hydrogen atom or a methyl group, R^{4b} represents a C₂₋₆ linear alkylene group, or a C₂₋₆ linear oxyalkylene group, and the linear alkylene group or the linear oxyalkylene group is optionally substituted with a C₁₋₆ alkyl group or a (meth)acryloyloxymethylene group in place of a hydrogen atom; and
wherein the core (X) is at least one selected from the group consisting of groups represented by the general formulas (5a) to (5h) below: 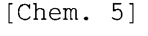 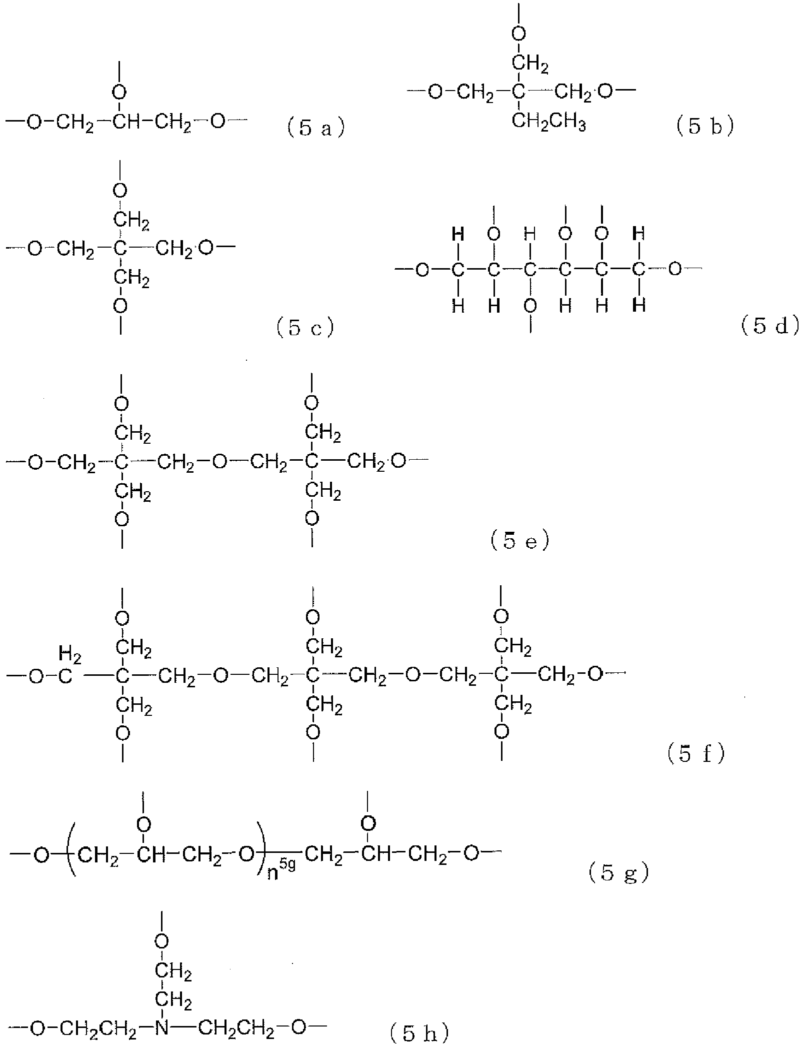
wherein in the general formula (5g), n^{5g} is an integer of 1 to 40.

2. The compound according to claim 1, wherein the terminal group (Y1) is a phosphorus-containing group represented by the general formula (2), a phosphorus-containing group represented by the general formula (3), a (meth)acryloyl group-containing group (Y2) represented by the general formula (4), or a hydrogen atom.

3. The compound according to claim 1 or 2, wherein the terminal group (Y1) is a phosphorus-containing group represented by the general formula (2), a phosphorus-containing group represented by the general formula (3), or a (meth)acryloyl group-containing group (Y2) represented by the general formula (4).

4. The compound according to any one of claims 1 to 3, wherein the (meth)acryloyl group-containing group (Y2) is at least one selected from the group consisting of groups represented by the general formulas (4a) to (4f) below.

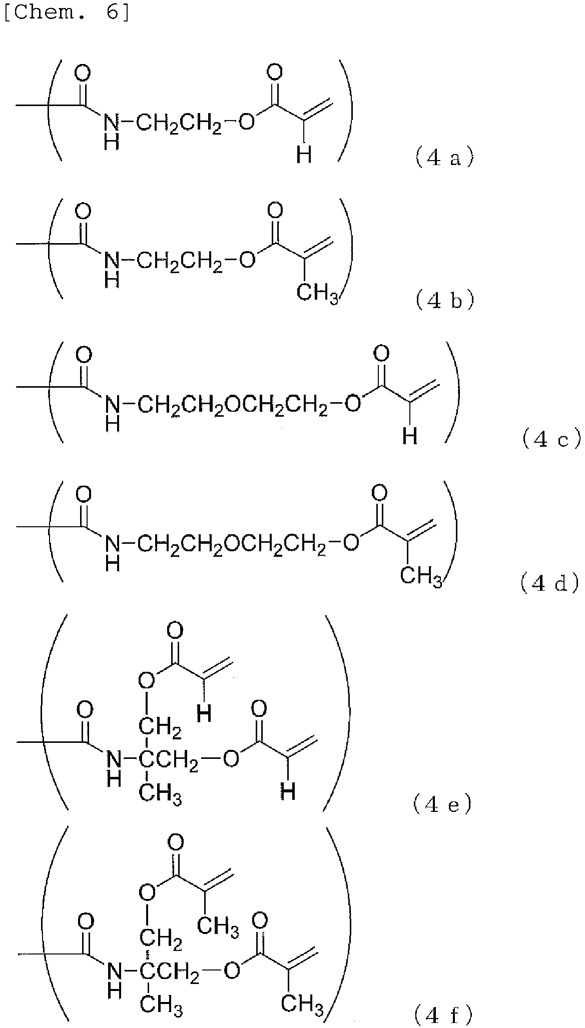

5. A composition comprising the compound according to any one of claims 1 to 4.

6. The composition according to claim 5, wherein the composition is negative in a reverse mutation test.

7. An adhesive material comprising the compound according to any one of claims 1 to 4.

8. The adhesive material according to claim 7, wherein the adhesive material is negative in a reverse mutation test.

9. A kit comprising the adhesive material according to claim 7 or 8.

## Patentansprüche

1. Verbindung der nachstehenden allgemeinen Formel (1), worin der nachstehende Kern (X) und die nachstehende endständige Gruppe (Y1) aneinander gebunden sind:
[Chem. 1]
X (Y1) n¹ (1)
wobei in der allgemeinen Formel (1) n¹ für die Anzahl der endständigen Gruppen (Y1) steht, die an den Kern (X) gebunden sind, und n¹ gleich der Wertigkeit des Kerns (X) ist; und
die endständige Gruppe (Y1) eine Phosphor-hältige Gruppe, die durch die nachstehende allgemeine Formel (2) dargestellt ist, eine Phosphor-hältige Gruppe, die durch die nachstehende allgemeine Formel (3) dargestellt ist, eine (Meth)acryloylgruppe-hältige Gruppe (Y2), die durch die nachstehende allgemeine Formel (4) dargestellt ist, eine (Meth)-acryloylgruppe, eine C₁₋₂₀-Kohlenwasserstoffgruppe oder ein Wasserstoffatom ist und eine Vielzahl an endständigen Gruppen (Y1) gleich oder unterschiedlich sein kann, mit der Maßgabe, dass unter allen endständigen Gruppen (Y1) in der durch die allgemeine Formel (1) dargestellten Verbindung eine oder mehrere endständige Gruppen Phosphor-hältige Gruppen der nachstehenden allgemeinen Formel (2) oder Phosphor-hältige Gruppen der nachstehenden allgemeinen Formel (3) sind und eine oder mehrere endständige Gruppen (Meth)-acryloylgruppe-hältige Gruppen (Y2) sind; 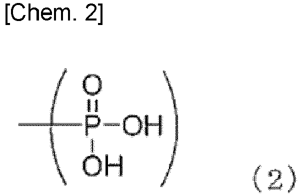 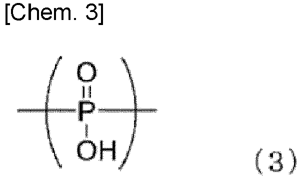
wobei in der allgemeinen Formel (3) ein Ende der Gruppe an den Kern (X) gebunden ist und das andere Ende der Gruppe an den Kern (X) gebunden ist, der in einer anderen Verbindung der allgemeinen Formel (1) vorhanden ist; und 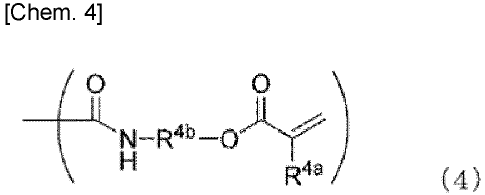
wobei in der allgemeinen Formel (4) R^{4a} für ein Wasserstoffatom oder eine Methylgruppe steht, R^{4b} für eine lineare C₂₋₆-Alkylengruppe oder eine lineare C₂₋₆-Oxyalkylengruppe steht und die lineare Alkylengruppe oder die lineare Oxyalkylengruppe gegebenenfalls mit einer C₁₋₆-Alkylgruppe oder einer (Meth)acryloyloxymethylengruppe anstelle eines Wasserstoffatoms substituiert ist; und
wobei der Kern (X) zumindest ein aus der aus Gruppen der nachstehenden Formeln (5a) bis (5h) bestehenden Gruppe ausgewählter ist: 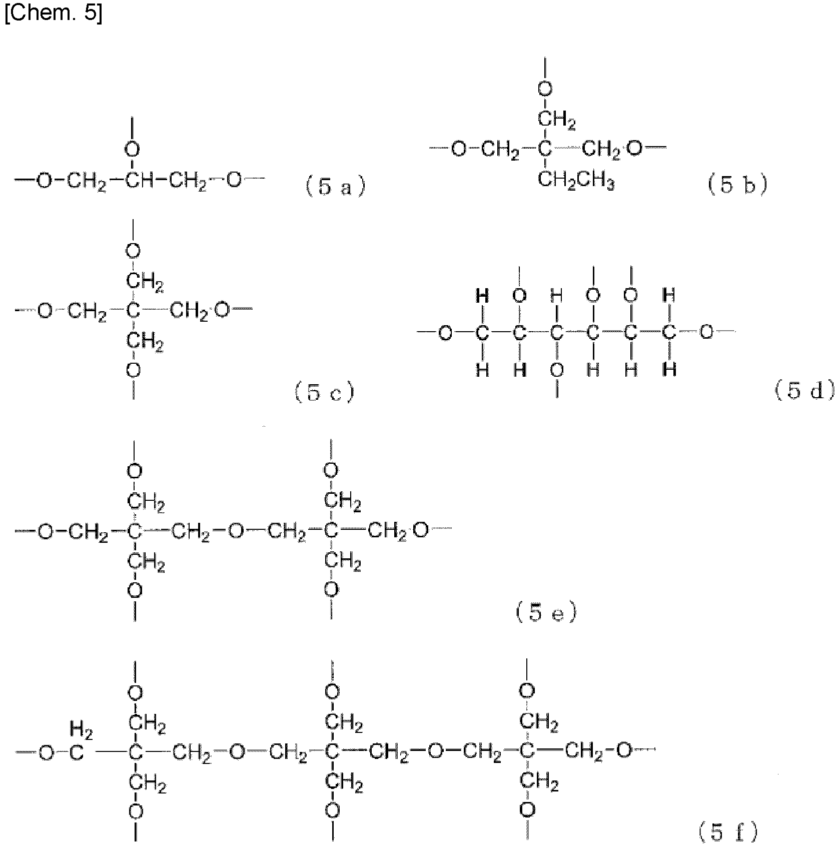 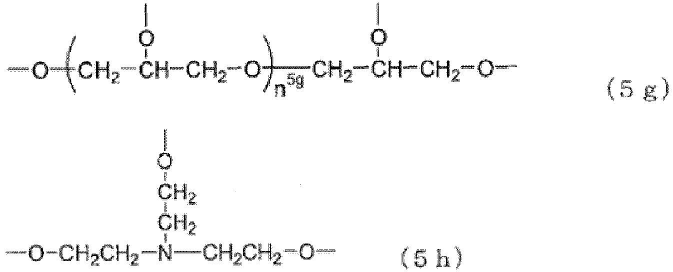
wobei in der allgemeinen Formel (5g) n^{5g} eine ganze Zahl von 1 bis 40 ist.

2. Verbindung nach Anspruch 1, wobei die endständige Gruppe (Y1) eine Phosphor-hältige Gruppe der allgemeinen Formel (2), eine Phosphor-hältige Gruppe der allgemeinen Formel (3), eine (Meth)acryloylgruppe-hältige Gruppe (Y2) der allgemeinen Formel (4) oder ein Wasserstoffatom ist.

3. Verbindung nach Anspruch 1 oder 2, wobei die endständige Gruppe (Y1) eine Phosphor-hältige Gruppe der allgemeinen Formel (2), eine Phosphor-hältige Gruppe der allgemeinen Formel (3) oder eine (Meth)acryloylgruppe-hältige Gruppe (Y2) der allgemeinen Formel (4) ist.

4. Verbindung nach einem der Ansprüche 1 bis 3, wobei die (Meth)acryloylgruppe-hältige Gruppe (Y2) zumindest eine aus der aus den Gruppen der nachstehenden allgemeinen Formeln (4a) bis (4f) bestehenden Gruppe ausgewählte ist.

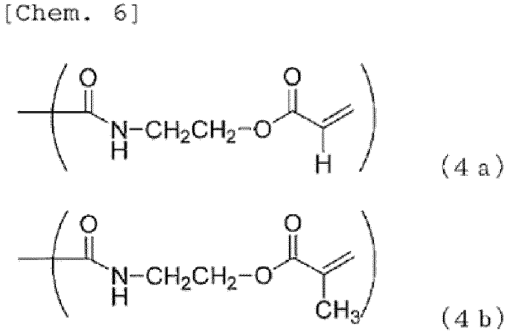

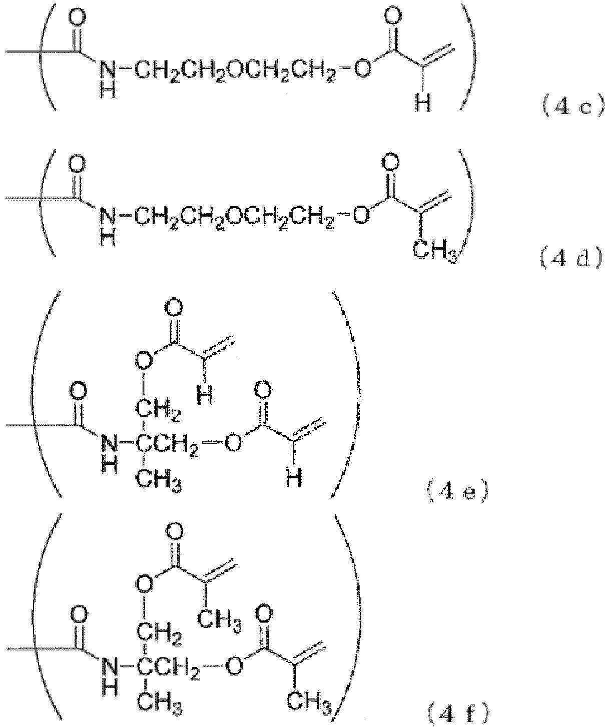

5. Zusammensetzung, die eine Verbindung nach einem der Ansprüche 1 bis 4 umfasst.

6. Zusammensetzung nach Anspruch 5, wobei die Zusammensetzung in einem Rückmutationstest negativ ist.

7. Klebematerial, die eine Verbindung nach einem der Ansprüche 1 bis 4 umfasst.

8. Klebematerial nach Anspruch 7, wobei das Klebematerial in einem Rückmutationstest negativ ist.

9. Set, das ein Klebematerial nach einem der Ansprüche 7 oder 8 umfasst.

## Revendications

1. Composé représenté par la formule générale (1) ci-dessous, dans laquelle le noyau (X) ci-dessous et le groupe terminal (Y1) ci-dessous sont liés l'un à l'autre :
[Chim. 1]
X (Y1) n¹ (1)
dans lequel dans la formule générale (1), n¹ représente le nombre de groupes terminaux (Y1) liés au noyau (X), et n¹ est égal à la valence du noyau (X) ; et
le groupe terminal (Y1) est un groupe contenant du phosphore représenté par la formule générale (2) ci-dessous, un groupe contenant du phosphore représenté par la formule générale (3) ci-dessous, un groupe contenant un groupe (méth)acryloyle (Y2) représenté par la formule générale (4) ci-dessous, un groupe (méth)acryloyle, un groupe hydrocarbure en C₁₋₂₀ ou un atome d'hydrogène, et une pluralité de groupes terminaux (Y1) peuvent être identiques ou différents les uns des autres, à condition que parmi tous les groupes terminaux (Y1) du composé représenté par la formule générale (1), un ou plusieurs groupes terminaux soient des groupes contenant du phosphore représentés par la formule générale (2) ci-dessous ou des groupes contenant du phosphore représentés par la formule générale (3) ci-dessous, et un ou plusieurs groupes terminaux soient des groupes contenant un groupe (méth)acryloyle (Y2) ; 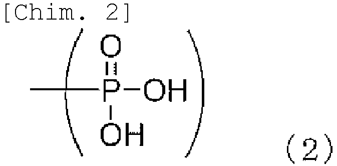 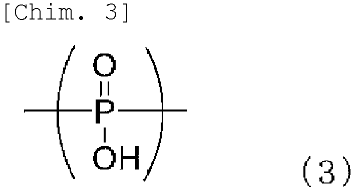
dans lequel dans la formule générale (3), une extrémité du groupe est liée au noyau (X), et l'autre extrémité du groupe est liée au noyau (X) présent dans un autre composé représenté par la formule générale (1) ; et 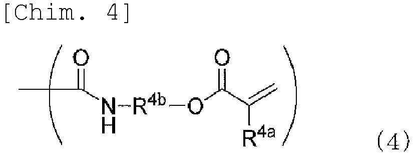
dans lequel dans la formule générale (4), R^{4a} représente un atome d'hydrogène ou un groupe méthyle, R^{4b} représente un groupe alkylène linéaire en C₂₋₆, ou un groupe oxyalkylène linéaire en C₂₋₆, et le groupe alkylène linéaire ou le groupe oxyalkylène linéaire est facultativement substitué par un groupe alkyle en C₁₋₆ ou un groupe (méth)acryloyloxyméthylène à la place d'un atome d'hydrogène ; et
dans lequel le noyau (X) est au moins un choisi dans le groupe constitué de groupes représentés par les formules générales (5a) à (5h) ci-dessous : 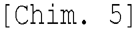 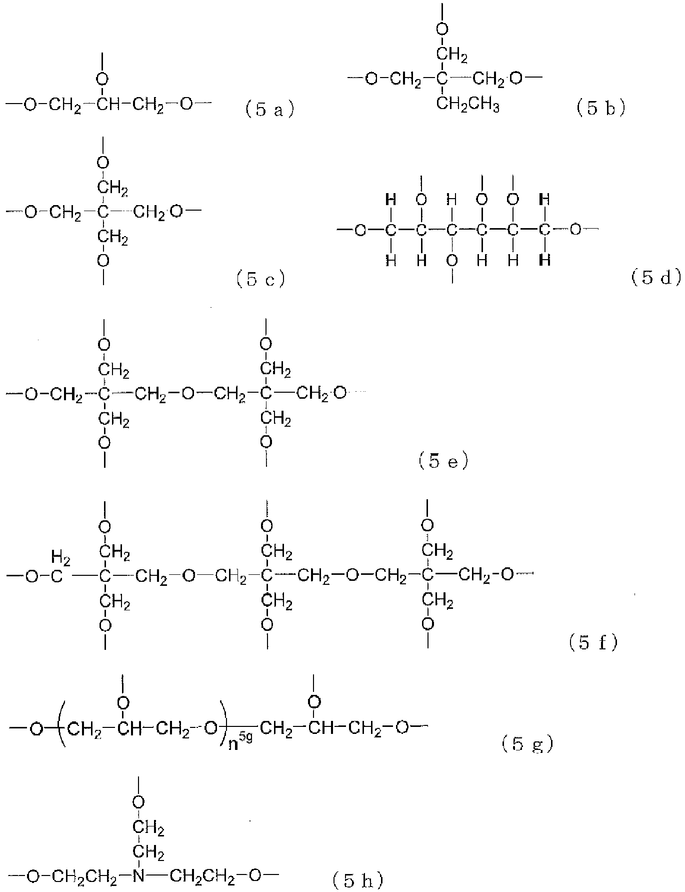
dans lequel dans la formule générale (5g), n^{5g} est un nombre entier de 1 à 40.

2. Composé selon la revendication 1, dans lequel le groupe terminal (Y1) est un groupe contenant du phosphore représenté par la formule générale (2), un groupe contenant du phosphore représenté par la formule générale (3), un groupe contenant un groupe (méth)acryloyle (Y2) représenté par la formule générale (4), ou un atome d'hydrogène.

3. Composé selon la revendication 1 ou 2, dans lequel le groupe terminal (Y1) est un groupe contenant du phosphore représenté par la formule générale (2), un groupe contenant du phosphore représenté par la formule générale (3), ou un groupe contenant un groupe (méth)acryloyle (Y2) représenté par la formule générale (4).

4. Composé selon l'une quelconque des revendications 1 à 3, dans lequel le groupe contenant un groupe (méth)acryloyle (Y2) est au moins un groupe choisi dans le groupe constitué des groupes représentés par les formules générales (4a) à (4f) ci-dessous.

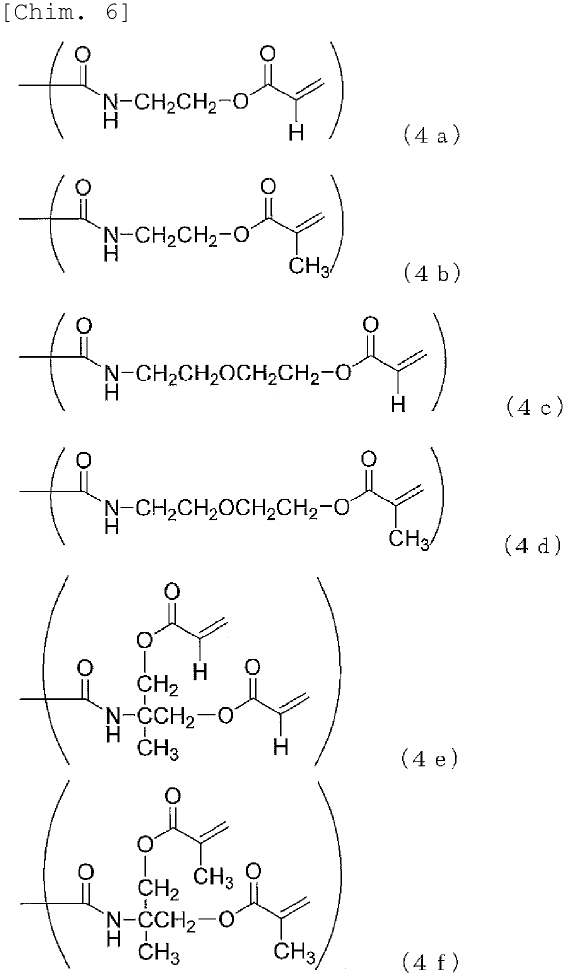

5. Composition comprenant le composé selon l'une quelconque des revendications 1 à 4.

6. Composition selon la revendication 5, **caractérisée en ce qu'**elle est négative lors d'un test de mutation inverse.

7. Matériau adhésif comprenant le composé selon l'une quelconque des revendications 1 à 4.

8. Matériau adhésif selon la revendication 7, dans lequel le matériau adhésif est négatif dans un test de mutation inverse.

9. Kit comprenant le matériau adhésif selon la revendication 7 ou 8.
